# EUROPEAN PATENT SPECIFICATION

(11) **EP 3 131 944 B1**
(45) Date of publication and mention of the grant of the patent: **05.12.2018**
(21) Application number: 15721401.6
(22) Date of filing: 17.04.2015
(51) Int. Cl.: A61K 9/00, C08G 65/332, A61K 47/10, A61K 47/22, A61K 9/10, A61K 47/34, A61K 9/107, A61K 31/337, A61K 31/713, C08G 65/331, C08G 65/333, C08G 65/334, C12N 15/113, A61K 31/727

(54) **VITAMIN E-BASED NANOCARRIERS FOR DRUG DELIVERY AND METHODS OF MAKING AND USING THE SAME**
NANOTRÄGER AUF VITAMIN-E-BASIS ZUR ARZNEIMITTELVERABREICHUNG UND VERFAHREN ZUR HERSTELLUNG DAVON
NANOVÉHICULES À BASE DE VITAMINE E POUR L'ADMINISTRATION DE MÉDICAMENTS ET LEURS PROCÉDÉS DE FABRICATION

(30) Priority: 17.04.2014 US 201461980786 P
(43) Date of publication of application: 22.02.2017
(73) Proprietor: The Corporation OF Mercer University, Macon, GA 31207 (US)
(72) Inventor: TAN, Chalet, Bogart, GA 30622 (US); FAN, Wei, Omaha, NE 68134 (US)
(74) Representative: Weickmann & Weickmann PartmbB
(86) International application number: PCT/US2015/026535
(87) International publication number: WO 2015/161295

(56) References cited:
- EP-A1- 1 891 962
- US-A1- 2008 187 568
- JIANQIN LU ET AL: "Design and Characterization of PEG-Derivatized Vitamin E as a Nanomicellar Formulation for Delivery of Paclitaxel", MOLECULAR PHARMACEUTICS, vol. 10, no. 8, 5 August 2013 (2013-08-05) , pages 2880-2890, XP055196703, ISSN: 1543-8384, DOI: 10.1021/mp300729y
- A. D. JENKINS et. al: "GLOSSARY OF BASIC TERMS IN POLYMER SCIENCE", Pure & Appl. Chem., vol. 68, no. 12 12 December 1996 (1996-12-12), pages 2287-2311, XP055196796, Retrieved from the Internet: URL:http://pac.iupac.org/publications/pac/ pdf/1996/pdf/6812x2287.pdf [retrieved on 2015-06-18]
- JUNTAO LUO ET AL: "Well-Defined, Size-Tunable, Multifunctional Micelles for Efficient Paclitaxel Delivery for Cancer Treatment", BIOCONJUGATE CHEMISTRY, vol. 21, no. 7, 21 July 2010 (2010-07-21), pages 1216-1224, XP055196709, ISSN: 1043-1802, DOI: 10.1021/bc1000033

## Description

This application is being filed as a PCT International Patent Application in the name of The Corporation of Mercer University, a U.S. corporation, claiming priority to U.S. Provisional Patent Application Serial No. 61/980,786 filed on 17 April, 2014 and entitled "VITAMIN E-BASED NANOCARRIERS FOR DRUG DELIVERY AND METHODS OF MAKING AND USING THE SAME."

### TECHNICAL FIELD

The present invention relates to vitamin E-based amphiphilic copolymers and compositions containing vitamin E-based amphiphilic copolymers and nanocarriers. The present invention also relates to methods of making vitamin E-based amphiphilic copolymers and nanocarriers, and methods of using vitamin E-based amphiphilic copolymers and nanocarriers, for example, as a drug delivery device.

### BACKGROUND

Jianqin Lu et al., Mol. Pharmaceutics 2013, 10, 2880-2890 describes PEG-derivatized Vitamin E as a nanomicellar formulation for delivery of paclitaxel. In particular, PTX-loaded PEG_{5K}-VE₂ was studied.

Juntao Luo et al., Bioconjugate Chem., Vol. 21, No. 7, 2010, 1216-1224 describes well-defined, size-tunable, multifunctional micelles for efficient paclitaxel delivery for cancer treatment. Described is an amphiphilic telodendrimer system being PEG-b-dendritic oligo-cholic acid.

US 2008/0187568 A1 describes a method for forming a crosslinked polymer hydrogel comprising: mixing a solution that comprises a medicinal agent dissolved in a water miscible solvent with a water soluble precursor to form a mixture and simultaneously precipitate the medicinal agent; depositing the mixture at a location exposed to a physiological fluid; and polymerizing the precursor to form the crosslinked hydrogel, entrap the precipitated agent, and subsequently gradually release the agent into the physiological fluid.

EP 1 891 962 A1 discloses a delivery system comprising a carrier or an active compound and a glutathione (GSH) or a glutathione derivative grafted thereon.

Polymerie micelles are versatile drug carrier systems that demonstrate great promise m the delivery of anticancer drugs. Currently, there are six micellar formulations for paclitaxel, doxorubicin and platinum drugs undergoing clinical trials and many more being explored in the preclinical studies. Composed of diverse amphiphilic copolymers, polymeric micelles are self-assembled supramolecular aggregates displaying typical core-shell architecture.
For example, D-α-Tocopherol polyethylene glycol succinate 1000 (TPGS_{1K}) is an amphiphilic copolymer generated by coupling PEG 1000 (PEG_{1K}) to Vitamin E (VE) succinate via an ester linkage. A safe pharmaceutical excipient approved by the FDA, TPGS_{1K} has been extensively investigated as an emulsifier, solubilizer and stabilizer in a variety of nano-sized drug delivery systems such as nanoparticles and micelles. With high lipophilicity conferred by the aliphatic chain and aromatic ring of the VE moiety, the inclusion of TPGS_{1K} in the mixed micelles not only greatly increases the loading capacity for hydrophobic drugs, but also stabilizes the micellar core by reducing the molecular motion of the copolymers. Nevertheless, as a micelle forming copolymer itself, TPGS_{1K} has an unfavorably high critical micelle concentration (CMC) at 0.2 g/L.
In a recent study, PEG was extended to 2 KDa and conjugated with VE at a 1:2 molar ratio, resulting in a PEG_{2K}-VE₂ copolymer, which formed micelles at a considerably lower CMC (1.14 mg/L), and could deliver doxorubicin with more potent anticancer efficacy than doxorubicin in the free form or in the TPGS_{1K} micelles. In another recent study, it was demonstrated that by further increasing PEG size to 5 KDa, the PEG_{5K}-VE₂ micelles (CMC ≈ 1.8 mg/L) functioned as an improved formulation for paclitaxel and achieved stronger tumor growth inhibition than Taxol® and paclitaxel-loaded PEG_{2K}-VE₂ micelles.

MicroRNAs (miRNAs) are endogenous small non-coding RNAs (∼22 nucleotides in length) that control gene expression at the posttranscriptional level by either inducing translation repression or promoting the degradation of messenger RNAs (mRNAs). By imperfectly basepairing with the 3'-untranslated region of the target mRNAs, each individual miRNA can concomitantly targets tens to hundreds of gene transcripts, regulating a large network of signaling pathways in the cells. It has been estimated that over 60% of all protein-coding genes are subject to miRNA regulation. As such, miRNAs are master regulators of gene expression essential to all aspects of the cellular processes.

Dysregulated miRNAs play causal roles in the onset, development and metastasis of cancer. Certain miRNAs have been recognized to function as tumor suppressors by directly downregulating oncogenes, and the expression such tumor suppressor miRNAs are commonly lost in tumor cells. MiRNA replacement therapy aims to restore tumor suppressor miRNAs in tumor cells to achieve anticancer effect. To reconstitute intracellular miRNA levels, one prevailing strategy is to employ miRNA mimics, which are synthetic doublestranded RNAs that contain the mature miRNA strands identical to the endogenous miRNAs. The first miRNA replacement therapy-MRX34-*miR*-*34a* mimic delivered by liposomal nanoparticles, has entered Phase I clinical trials for the treatment of hepatocellular carcinoma since 2013.

*Let-7* is a family of tumor suppressor miRNAs crucially involved in cell differentiation, proliferation and motility by directly targeting pleotropic oncogenes including RAS, MYC, *HMGA2, GAB2* and *FN1* among others. Abundantly expressed in the healthy tissues, *let-7* is frequently suppressed in solid tumors and is associated with poor prognosis in non-small cell lung cancer (NSCLC), breast and gastric cancers. Repletion of *let-7* has been shown to suppress tumor initiation in the mouse models of NSCLC. However, delivery of *let-7* mimic or *let*-*7-*encoding plasmids to the pre-established tumors exhibits limited anticancer potency owing to the inability of *let-7* to trigger apoptosis in tumor cells.

Efforts continue to further develop effective drug carrier systems capable of effectively delivering drugs within a patient, such as anticancer drugs to a tumor location within a human or other animal.

### SUMMARY

The present invention addresses some of the difficulties and problems discussed above by the discovery of new amphiphilic copolymers and nanocarriers formed therefrom, both of which have exceptional drug association/complexing properties, as well as complex stability.

Accordingly, the present invention is directed to amphiphilic copolymers. The amphiphilic copolymers of the present invention comprise: polyethylene glycol, and three or more vitamin E units bonded (i.e., covalently bonded directly or indirectly) to the polyethylene glycol.

The amphiphilic copolymer comprises three or more vitamin E units bonded (directly or indirectly) to the polyethylene glycol. Typically, the amphiphilic copolymer comprises from four to eight vitamin E units bonded to the polyethylene glycol.

Each vitamin E unit may be bonded to the polyethylene glycol via one or more divalent linkages. Typically. the one or more divalent linkages comprise one or more divalent linkages covalently bonded between each vitamin E unit and the polyethylene glycol. In addition to the one or more divalent linkages, the amphiphilic copolymers (and nanocarriers formed therefrom) of the present invention may further comprise one or more crosslinking moieties bonded to the polyethylene glycol and/or the one or more divalent linkages.

The present invention is further directed to compositions comprising the herein-described amphiphilic copolymers and/or nanocarriers. In one exemplary embodiment the composition of the present invention comprises one or more amphiphilic copolymers, as described herein (or nanocarriers formed therefrom), and at least one biologically active substance incorporated within the one or more amphiphilic copolymers (or nanocarriers formed therefrom). Suitable biologically active substances include, but are not limited to. a drug, an anti-carcinogenic compound, a nucleic acid, another small molecule, or any combination thereof. In one desired embodiment, the biologically active substance comprises an anti-carcinogenic compound. Suitable anti-carcinogenic compounds include, but are not limited to, paclitaxel, docetaxel. carbazitaxel, ixabepilone, eribulin, topotecan. irinotecan, SN-38, doxorubicin, daunorubicin. idarubicin, epirubicin, etoposide and omacetaxine. Suitable nucleic acids include, but are not limited to, miRNA mimics (e.g., let-7 mimic, and miR-34 mimic), anti-miRs, siRNAs and antisense oligonucleotides. The compositions may further comprise one or more additional components including, but not limited to, combinations of two or more of the herein-described nanocarriers, water, sodium phosphate, sodium chloride, glucose, HEPES (i.e., 2-[4-(2-hydroxyethyl)piperazin-1-yl]ethanesulfonic acid) and mannitol.

The present invention is even further directed to methods of making amphiphilic copolymers, nanocarriers and compositions containing the same. In one exemplary embodiment, the method of making an amphiphilic copolymer comprises covalently bonding three or more vitamin E units to polyethylene glycol. The methods of making amphiphilic copolymers, nanocarriers, or compositions containing the same, may further comprise one or more steps including, but not limited to, reacting a functionalized polyethylene glycol having a terminal amine end group and a terminal carboxyl end group with one or more divalent linkages; reacting functional groups on the one or more divalent linkages with the three or more vitamin E units; reacting one or more crosslinking moieties with either the polyethylene glycol, the one or more divalent linkages, or both; combining at least one biologically active substance with the amphiphilic copolymer or nanocarrier; and incorporating (i) the biologically active substance and (ii) the amphiphilic copolymer and/or nanocarrier into an aqueous solution.

These and other features and advantages of the present invention will become apparent after a review of the following detailed description of the disclosed embodiments and the appended claims.

### BRIEF DESCRIPTION OF THE FIGURES

FIG. **1** depicts a schematic illustration of the design and action of exemplary crosslinked PEG_{5K}-VE₄-TA₄ micelles;
FIGS. **2A-2C** depict 1H NMR spectra of intermediate 5 (A), 6 (B) and the PEG_{5K}-VE₄-TA₄ copolymer (C) in CDCl₃;
FIGS. **3A-3F** depict size distribution and zeta potential of P-NCMs (A and C) and P-CMs (B and D), and TEM images of P-NCMs (E) and P-CMs (F) (Scale bars: 100 nm);
FIGS. **4A-4D** graphically depict stability of paclitaxel-loaded PEG_{5K}-VE₄-TA₄ micelles in different solutions: (A) the size and paclitaxel concentration of P-NCMs or P-CMs in PBS, (B) the size distribution of P-NCMs and P-CMs in PBS containing 2.5 mg/mL SDS, (C) the size distribution of P-NCMs and P-CMs in PBS containing 50% FBS, and (D) the size distribution of P-CMs in PBS containing 2 µM or 10 mM GSH (Note, all results show representative data obtained from at least three independent experiments);
FIGS. **5A-5B** graphically depict (A) the release of paclitaxel from P-NCMs and P-CMs, and (B) the release of paclitaxel from P-CMs in the absence or presence of GSH (Note, all results show representative data obtained from at least three independent experiments and are reported as the mean ± SD (n=3));

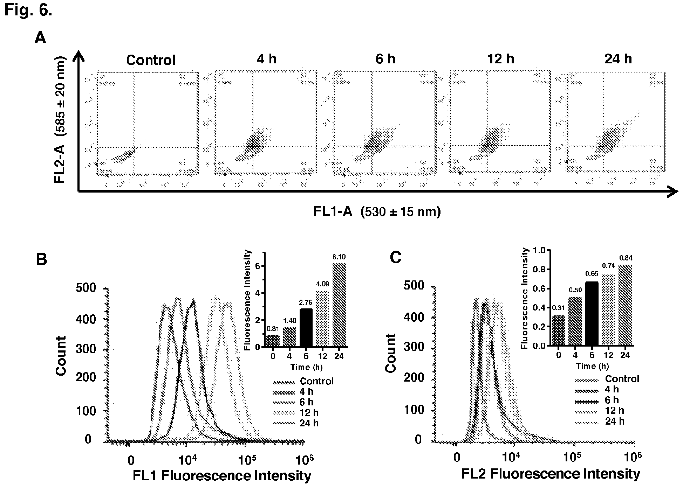

FIGS. **6A-6C** graphically depict cellular uptake of the crosslinked PEG_{5K}-VE₄-TA₄ micelles in SKOV-3 cells (Note, cells were incubated with DiO/DiI dual loaded crosslinked micelles for up to 24 h. The intracellular fluorescence intensity was analyzed by a flow cytometer, as displayed in dot plots (A) and histogram plots (B, C). The inserts show the median fluorescence intensity (MFI) of each cell population. All results show representative data obtained from three independent experiments. The excitation wavelength was set at 488 nm, the FL1 channel had a spectral filter of 530 ± 15 nm to detect the fluorescence emission from DiO, and the FL2 channel with a spectral filter of 585 ± 20 nm to detect the fluorescence emission from DiI.);
FIGS. **7A-7B** graphically depict (A) both the crosslinked and non-crosslinked PEG_{5K}-VE₄-TA₄ micelles are minimally cytotoxic in SKOV-3 cells at the copolymer concentration up to 10 g/L, and (B) the cytotoxicity of paclitaxel in the free form, or in the crosslinked or non-crosslinked PEG_{5K}-VE₄-TA₄ micelles against SKOV-3 cells (Note, results show representative data obtained from at least three independent experiments and are reported as the mean ± SD (n≥3).);
FIG. **8** graphically depicts the plasma concentration of paclitaxel as a function of time following intravenous administration of Taxol®, P-NCMs and P-CMs at an identical dose of 5 mg/kg paclitaxel (Note, each data point represents the mean ± SD (n = 3).);
FIGS. **9A-9C** depict (A) whole-body NIR imaging of SKOV-3 tumor-bearing mice treated with DiD/paclitaxel-loaded PEG_{5K}-VE₄-TA₄ micelles for 48 h (Note, DP-CMs and DP-NCMs were i.v. injected in mice at an identical dose with respect to DiD and paclitaxel.), (B) NIR imaging of the normal organs and tumors excised at 24 h and 48 h post injection, and (C) quantitative measurement of the DiD intensity in the normal organs and tumors (Note, values are normalized by the scan area and reported as the mean + SD (n=3).);
FIGS. **10A-10C** depict (A) antitumor efficacy of paclitaxel-loaded PEG_{5K}-VE₄-TA₄ micelles in mice bearing SKOV-3 tumors (Note, P-NCMs, P-CMs and Taxol® were i.v. administered to the mice at 5 mg/kg paclitaxel twice-weekly for 3 weeks. B-CMs, P-NCMs and P-CMs were prepared with an identical polymer concentration.), (B) images of the tumors dissected from the tumor-bearing mice on day 35, and (C) the average body weight of the mice in all groups (Note, the results represent the mean ± SD (n=5), P < 0.01.);
FIGS. **11A-11B** depict (A) H&E staining and immunohistochemistry staining for PCNA of the tumor sections, Scale bars: 200 µm in the large images and 100 µm in the small inserts, and (B) the protein levels of PCNA and p-AKT in the tumor lysates by Western blot analysis (Note, β-actin was used as a loading control. All results show representative data of the tumor tissue samples.);
FIG. **12** depicts an exemplary reaction scheme for preparing an exemplary vitamin E-based nanocarrier of the present invention;
FIGS. **13A-13B** graphically depict the UV spectra of B-NCMs (A) and B-CMs (B);
FIGS. **14A-14B** graphically depict the effect of the input concentration of paclitaxel on the drug loading (A) and the average size (B) of P-NCMs and P-CMs.
FIGS. **15A-15B** graphically depict the critical micelle concentration (CMC) of the non-crosslinked PEG_{5K}-VE₄-TA₄ micelles (A) and the crosslinked PEG_{5K}-VE₄-TA₄ micelles (B);
FIG. **16** graphically depict cellular uptake of DiO, DiI or DiO/DiI-loaded crosslinked PEG_{5K}-VE₄-TA₄ micelles in SKOV-3 cells;
FIGS. **17A-17B** depict exemplary reaction schemes for preparing exemplary vitamin E-based nanocarriers of the present invention;
FIGS. **18A-18C** depict 1H NMR spectra of PEG_{5K}-VE₄ (A), the intermediate 7 shown in FIG. **17A** (B) and PEG_{5K} -VE₄-DET₂₀ (C) in CDCl₃;
FIGS. **19A-19E** depict electrophoretic retardation of *let-7b* mimic (50 pmol) binding by blank micelles (A) and paclitaxel-loaded micelles (B) at different N/P ratios, heparin dissociation assay of *let-7b* mimic (50 pmol) from the blank micelles (C) and the paclitaxel-loaded micelles (D) at different miRNA:heparin weight ratio, and stability of *let-7b* mimic-loaded micelleplexes in 10% FBS at 37 °C (E);
FIGS. **20A-20C** depict size distribution, zeta potential and TEM images of blank micelles (A), paclitaxel-loaded micelles (i.e., M-PTX) (B) and paclitaxel- and *let-7b-* loaded micelles (i.e., M-PTX/*let-7b*) (C);
FIGS. **21A-21B** depict the release of paclitaxel (A) and *let-7b* mimic (B) from M-*PTX*/*let-7b* at pH 7.4 and 5.5 in the absence or presence of cathepsin B;
FIGS. **22A-22C** depict (A) confocal fluorescence images of A549 cells treated by Oregon green-labeled paclitaxel/Cy3-labeled *let-7b* mimic-dual loaded micelles for up to 48 h. with (i) LysoTraker stained lysosomes shown in red, (ii) Oregon green-labeled paclitaxel shown in green, and (iii) Cy3-labeled let-7b shown in yellow; (B) *let-7b* level in cells transfected by M-PTX/*let-7b* at different N/P ratios with lipofectamine 2000 as a positive control; and (C) expression of KRAS protein in cells transfected by M-PTX/*let-7b* at different N/P ratios;
FIGS. **23A-23D** depict (A) cytotoxicity of the blank micelles in A549 cells; (B) cytotoxicity of free paclitaxel, M-PTX and M-PTX/*let-7b* against A549 cells, (C) effect of the blank micelles, M-PTX, *M-let-7b* and M-PTX/*let-7b* on the colony formation of A549 cells, and (D) effect of M-PTX/*let-7b* on the phosphorylation of AKT and ERK1/2;
FIGS. **24A-24C** depict (A) effect of *M-PTX*/*let-7b* on apoptosis in A549 cells detected by flow cytometry using Annexin V and PI staining, (B) quantification of late apoptotic cells (Annexin⁺/PI⁺ cells), and (C) induction of cleaved caspase-3 and cleaved PARP as apoptotic marker proteins in A549 cells;
FIGS. **25A-24C** depict (A) effect of M-PTX/*let-7b* on the invasion capacity of A549 cells (Images were acquired using an inverted microscope at × 40 magnification.); (B) quantification of the invading cell population; ad (C) effect of M-PTX/*let-7b* on the protein expression of HMGA2, E-cadherin and vimentin in A549;
FIGS. **26A-26C** depict (A) antitumor efficacy of M-PTX/*let-7b* in mice bearing A549 tumors following intravenous administration; (B) images of tumors dissected on day 42; and (C) average body weight of mice in all groups;
FIGS. **27A-27D** depict (A) expression of *let-7b* in tumor tissues quantified by qRT-PCR, with U6 used as an internal control; (B) protein levels of KRAS, p-AKT, AKT and PCNA in tumor tissues with β-actin used as a loading control; and IHC staining for PCNA (C) and H&E staining (D) of the tumor sections (Images were acquired at × 40 magnification.);
FIG. **28** depicts CMC of PEG_{5K}-VE₄, PEG_{5K}-VE₄-DET₂₀ and the hybrid micelles; and
FIGS. **29A-29B** depict stability of the PEG_{5K}-VE₄-DET₂₀ hybrid micelles in PBS as reflected by the change in the size (A) and the loading concentration of paclitaxel (B).

### DETAILED DESCRIPTION

To promote an understanding of the principles of the present invention, descriptions of specific embodiments of the invention follow and specific language is used to describe the specific embodiments. It will nevertheless be understood that no limitation of the scope of the invention is intended by the use of specific language. Alterations, further modifications, and such further applications of the principles of the present invention discussed are contemplated as would normally occur to one ordinarily skilled in the art to which the invention pertains.

The present invention is directed to amphiphilic copolymers and nanocarriers comprising a reaction product of PEG and vitamin E, as well as compositions containing the same. The present invention is further directed to methods of making amphiphilic copolymers, nanocarriers and compositions containing a reaction product of PEG and Vitamin E.

The amphiphilic copolymers of the present invention utilize (i) polyethylene glycol (PEG) as a hydrophilic block, which shields the amphiphilic copolymer (and nanoconstructs formed therefrom) and confers a stealth property by sterically hindering protein adsorption, and (ii) a tailored hydrophobic segment in the form of multiple Vitamin E units (i.e., derived from α-tocopheryloxyacetic acid) to optimize encapsulation of a nanocarrier payload (e.g., a drug). The disclosed micellar nanocarriers enable a tunable loading capacity for various hydrophobic drugs within the highly lipophilic micellar core via physical entrapment, which alleviates the use of organic solvents and greatly increases the aqueous solubility of otherwise water-insoluble drugs.

Another potential advantage of the herein-disclosed polymeric micelles (i.e., nanocarriers) for cancer therapy resides in their nanoscale dimensions, which can allow for preferential accumulation of anticancer drugs in the tumor via the enhanced permeability and retention (EPR) effect owing to leaky vasculature and diminished lymphatic drainage in solid tumors. To realize this benefit, it is desirable that the nanocarrier micelles maintain structural integrity and retain the payload for sufficient duration while circulating in the bloodstream. Although thermodynamic and kinetic instability of micelles *in vivo* poses significant challenges to minimize the burst release of a given payload before the drug-loaded micelles arrive at the tumor site, the nanocarrier micelles of the present invention exhibit exceptional stability when tested in *in vivo* conditions (as discussed in the Example section below).

To reinforce structural integrity of the nanocarrier polymeric micelles of the present invention and assist these self-assembled aggregates so as to withstand the destabilizing environment *in vivo,* adjacent polymeric chains of the nanocarrier polymeric micelles may be covalently crosslinked to generate a single, nanostructured macromolecule. Various chemical reactions, such as radical polymerization, UV crosslinking, disulfide linkage, polyelectrolyte complexation, silicon chemistry and click chemistry, may be used to form the crosslinked micelles. By conjugating the polymer backbone with cross-linkable moieties or introducing external bifunctional crosslinkers, cross-linkages may be formed at the shell, core or core-shell intermediate region of the herein-disclosed nanocarrier micelles. For the herein-disclosed crosslinked nanocarrier micelles to serve as biocompatible and biodegradable drug carriers, it is desirable for the crosslinking reactions to occur in an aqueous solution, employ/produce non-toxic substances, and be readily reversible in the tumor to facilitate the release of the payload and the elimination of the polymers.

In some embodiments, the amphiphilic copolymers and nanocarriers of the present invention utilize disulfide linkage crosslinking, which demonstrates excellent reversibility and biocompatibility. For example, cysteine-containing copolymers may be utilized to install free thiol groups onto the nanocarrier polymer chains, so that disulfide-crosslinked nanocarrier micelles can be generated under mildly oxidative conditions. Alternatively, external disulfide-containing linkers may be used to produce the crosslinked nanocarrier micelles. In other embodiments, thioctic acid (TA), an endogenous metabolite and a naturally occurring antioxidant, may be grafted onto the amphiphilic copolymer backbone as a crosslinker. The disulfide-containing thioctic ring is susceptible to ring opening polymerization in the presence of a catalytic amount of dithiothreitol (DTT), which results in the formation of linear polydisulfide crosslinks among adjacent polymer chains within the nanocarrier micelles. As delivery vehicles for hydrophobic chemotherapeutic drugs including paclitaxel, docetaxel and doxorubicin, the disulfide-crosslinked micelles demonstrate improved stability in the circulation and enhanced antitumor efficacy in murine xenograft models.

Further, disulfide crosslinks are redox-responsive and prone to cleavage through thioldisulfide exchange reactions with glutathione (GSH), an endogenous reducing agent present at much higher level in the cytosol of tissue cells (1-10 mM) than in plasma and the interstitial fluid (2-10 µM). GSH level is found to be further elevated in the tumor cells, rendering disulfide-crosslinked nanocarrier micelles particularly suitable for intratumor delivery of anticancer drugs.

### I. Amphiphilic Copolymers and Nanocarriers - Reaction Product of PEG and Vitamin E

As discussed herein, amphiphilic copolymers and nanocarriers of the present invention comprise a reaction product of PEG and multiple Vitamin E units. Other amphiphilic copolymers and nanocarriers of the present invention comprise a reaction product of, *inter alia,* PEG, multiple Vitamin E units, one or more divalent linkages, and one or more moieties such as a crosslinking moiety or a cationic moeity. The amphiphilic copolymers and nanocarriers of the present invention are further described in the embodiments below.

### Amphiphilic Copolymers. Nanocarriers and Compositions Embodiments:

1. An amphiphilic copolymer comprising: polyethylene glycol, and three or more vitamin E units bonded to the polyethylene glycol.
2. A crosslinking amphiphilic copolymer comprising: polyethylene glycol, and two or more vitamin E units bonded to the polyethylene glycol. As used herein, the term "crosslinking amphiphilic copolymer" refers to amphiphilic copolymers in which at least a portion of the amphiphilic polymer is crosslinkable with itself and/or other amphiphilic copolymers (not an embodiment of the invention).
3. The amphiphilic copolymer of embodiment 2, wherein said amphiphilic copolymer comprises three or more vitamin E units bonded to the polyethylene glycol.
4. The amphiphilic copolymer of any one of embodiments 1 to 3, wherein said amphiphilic copolymer comprises from four to eight vitamin E units bonded to the polyethylene glycol. It should be understood that the term "said amphiphilic copolymer" or "the amphiphilic copolymer" used in the embodiments below refers to both the amphiphilic copolymer recited in embodiment 1 and the crosslinking amphiphilic copolymer recited in embodiment 2.
5. The amphiphilic copolymer of any one of embodiments 1 to 4, wherein said amphiphilic copolymer comprises four vitamin E units bonded to the polyethylene glycol.
6. The amphiphilic copolymer of any one of embodiments 1 to 5, wherein said polyethylene glycol has a molecular weight of from about 1000 to 500,000 (or any molecular weight between 1000 and 500,000. e.g., 5000, or any range of values between 1000 and 500,000. e.g., 4500-5500, in increments of 1.0).
7. The amphiphilic copolymer of any one of embodiments 1 to 6, wherein said polyethylene glycol has a molecular weight of from 5000 to 20,000.
8. The amphiphilic copolymer of any one of embodiments 1 to 7, wherein each vitamin E unit is bonded to said polyethylene glycol via one or more divalent linkages.
9. The amphiphilic copolymer of any one of embodiments 1 to 8, said amphiphilic copolymer having a structure: 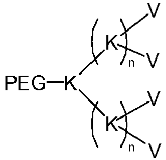 wherein PEG represents said polyethylene glycol, V represents a given vitamin E unit, K represents a first divalent linkage, and independently each n = 0 to 5.
10. The amphiphilic copolymer of any one of embodiments 1 to 9, said amphiphilic copolymer having a structure: 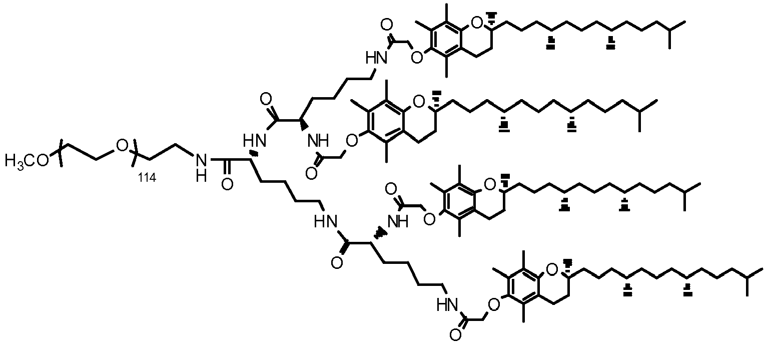
11. The amphiphilic copolymer of any one of embodiments 1 to 8, said amphiphilic copolymer having a structure: 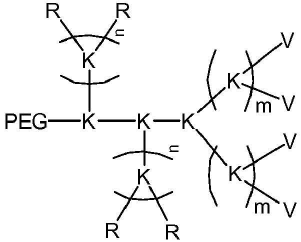 wherein PEG represents said polyethylene glycol, V represents a given vitamin E unit, K represents a first divalent linkage, R represents a crosslinking or cationic moiety, independently each m = 0 to 5, and independently each n = 0 to 20.
12. The amphiphilic copolymer of any one of embodiments 1 to 8 and 11, said amphiphilic copolymer having a structure: 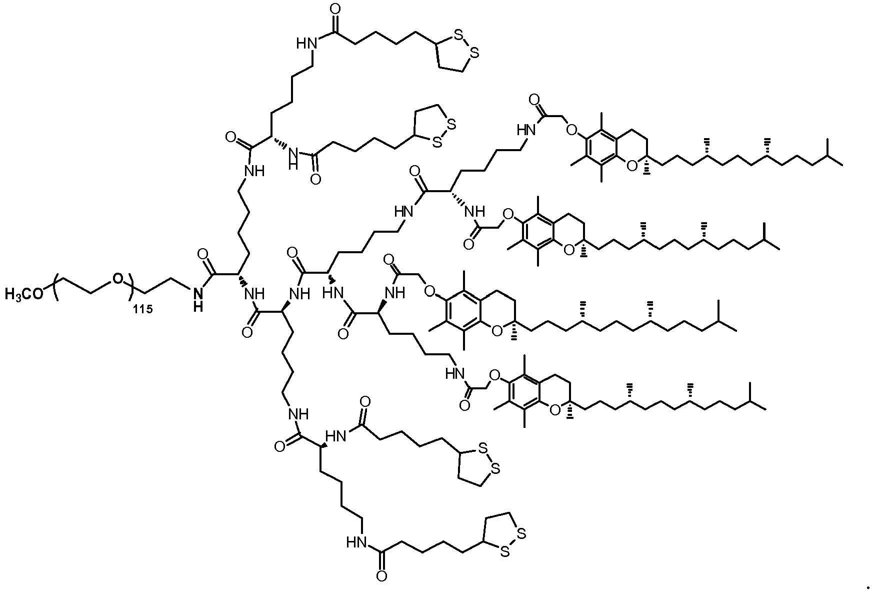
13. The amphiphilic copolymer of any one of embodiments 1 to 8, said amphiphilic copolymer having a structure: 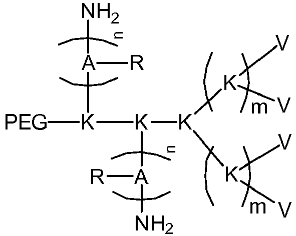 wherein PEG represents said polyethylene glycol, V represents a given vitamin E unit, K represents a first divalent linkage, A represents a second divalent linkage, R represents a crosslinking or cationic moiety, independently each m = 0 to 5, and independently each n = 0 to 20.
14. The amphiphilic copolymer of any one of embodiments 1 to 8 and 13, said amphiphilic copolymer having a structure: 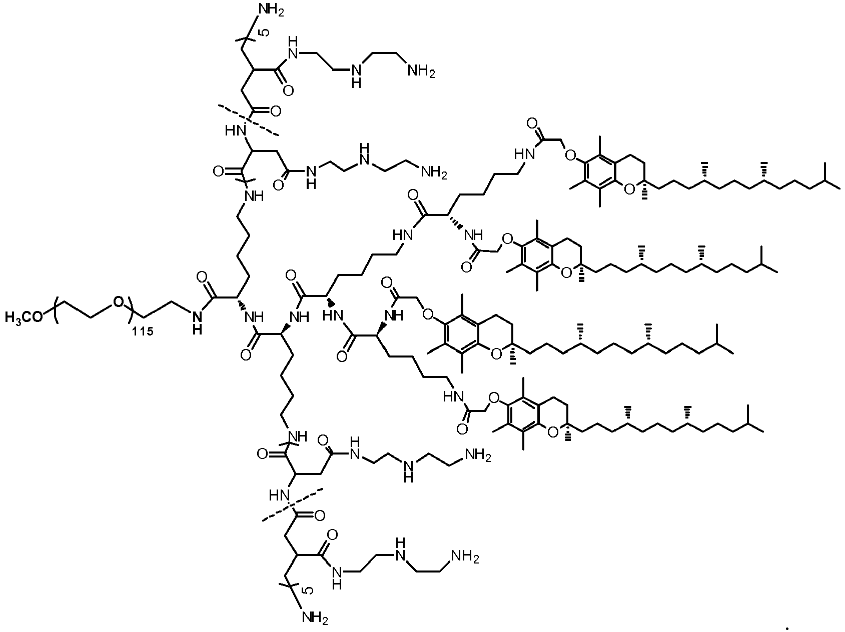
15. The amphiphilic copolymer of any one of embodiments 1 to 8, said amphiphilic copolymer having a structure: 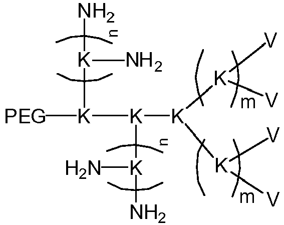 wherein PEG represents said polyethylene glycol, V represents a given vitamin E unit, K represents a first divalent linkage, independently each m = 0 to 5, and independently each n = 0 to 20.
16. The amphiphilic copolymer of any one of embodiments 1 to 8 and 15, said amphiphilic copolymer having a structure: 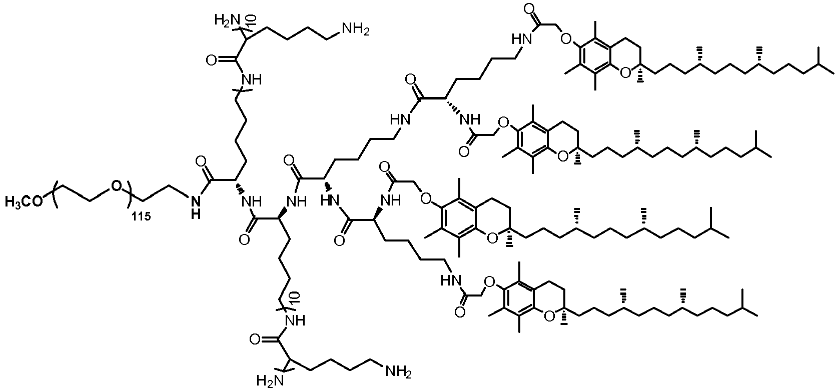
17. The amphiphilic copolymer of any one of embodiments 1 to 8, said amphiphilic copolymer having a structure: 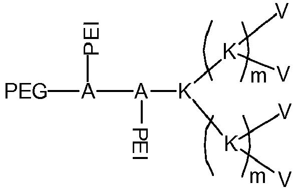 wherein PEG represents said polyethylene glycol, V represents a given vitamin E unit, K represents a first divalent linkage, A represents a second divalent linkage, PEI represents a polyethylenimine unit, and independently each m = 0 to 5.
18. The amphiphilic copolymer of any one of embodiments 1 to 8 and 17, said amphiphilic copolymer having a structure: 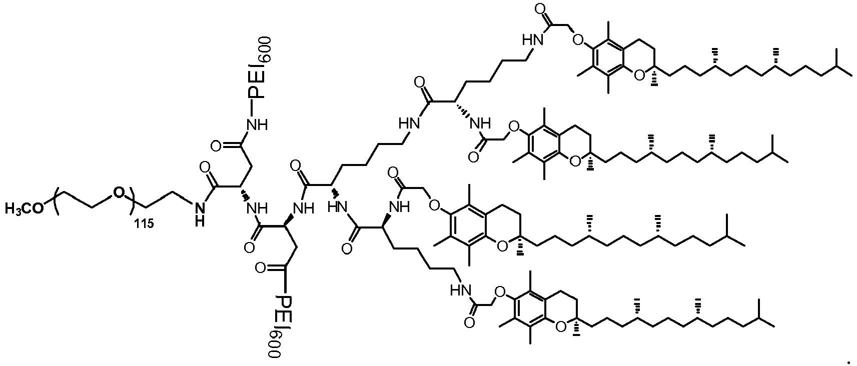
19. The amphiphilic copolymer of any one of embodiments 8 to 18, wherein each of said one or more divalent linkages independently comprises lysine, N_{α}-Fmoc-N_{ε}-Boc-L-lysine, or aspartic acid.
20. The amphiphilic copolymer of any one of embodiments 8 to 19, wherein each K comprises lysine or N_{α}-Fmoc-N_{ε}-Boc-L-lysine.
21. The amphiphilic copolymer of any one of embodiments 8 to 20, wherein each K comprises N_{α}-Fmoc-N_{ε}-Boc-L-lysine.
22. The amphiphilic copolymer of any one of embodiments 13 to 14 and 17 to 21, wherein each A comprises aspartic acid or β-benzyl-L-aspartate N-carboxy anhydride.
23. The amphiphilic copolymer of any one of embodiments 13 to 14 and 17 to 22, wherein each A comprises β-benzyl-L-aspartate N-carboxy anhydride.
24. The amphiphilic copolymer of any one of embodiments 1 to 8, 13 and 19 to 23, said amphiphilic copolymer having a structure: 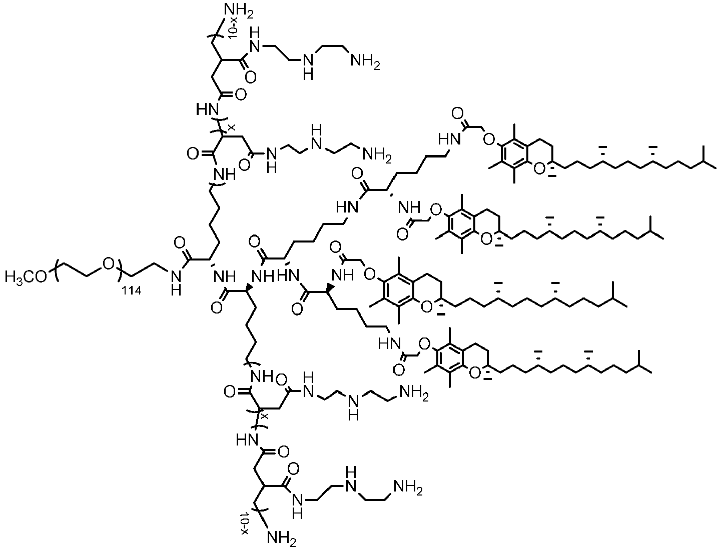 wherein x equals a number from 0 to 10.
25. The amphiphilic copolymer of any one of embodiments 9 to 24, wherein each V comprises α-tocopheryloxyacetic acid (i.e., each V comprises α-tocopheryloxyacetic acid minus an -OH group).
26. The amphiphilic copolymer of any one of embodiments 1 to 25, wherein each vitamin E unit comprises α-tocopheryloxyacetic acid (i.e., each vitamin E unit comprises α-tocopheryloxyacetic acid minus an -OH group).
27. The amphiphilic copolymer of any one of embodiments 11 to 14 and 19 to 26, wherein each R independently comprises thioctic acid, cysteine, diethylenetriamine, triethylenetetramine, tris(2-aminoethyl)amine or N,N-diisopropylethylenediamine (i.e., each R group represents a given R group minus one or more atoms removed from the R group so as to bond with the PEG polymer chain of the nanocarrier, e.g., an -OH group for thioctic acid).
28. The amphiphilic copolymer of any one of embodiments 11 to 14 and 19 to 27, wherein each R independently comprises diethylenetriamine, triethylenetetramine, tris(2- aminoethyl)amine or N,N-diisopropylethylenediamine. In some preferred embodiments, each R comprises diethylenetriamine.
29. The amphiphilic copolymer of any one of embodiments 17 to 20, 22 and 25 to 27, wherein each PEI independently comprises a polyethylenimine unit having a molecular weight ranging from 200 to 2500 (or any value between 200 and 2500, e.g., 600, or any range of values between 200 and 2500, e.g., 600-650, in increments of 1 .0).
30. The amphiphilic copolymer of any one of embodiments 9 to 29, wherein each PEG is represented by:

   H₃CO-(CH₂CH₂O)_{z},-CH₂CH₂NH-

   wherein z ranges from 25 to 12,500 (or any value between 25 and 12,500, e.g., 115, or any range of values between 25 and 12,500, e.g., 100-137, in increments of 1.0).
31. The amphiphilic copolymer of embodiment 30, wherein z ranges from 100 to 125.
32. The amphiphilic copolymer of any one of embodiments 2 to 31, wherein said crosslinking amphiphilic copolymer comprises one or more crosslinking moieties bonded (directly or indirectly) to said polyethylene glycol, wherein each crosslinking moiety independently comprises thioctic acid and cysteine.
33. The amphiphilic copolymer of any one of embodiments 2 to 32, wherein said crosslinked amphiphilic copolymer comprises one or more crosslinking moieties bonded (directly or indirectly) to said polyethylene glycol, wherein each crosslinking moiety comprises thioctic acid.
34. A nanocarrier comprising one or more amphiphilic copolymers, wherein each amphiphilic copolymer independently comprises the amphiphilic copolymer of any one of embodiments 1 to 33.
35. The nanocarrier of embodiment 34, wherein said nanocarrier has an average particle size ranging from 10 nm to 1000 nm (or any value between 10 and 1000 nm, e.g., 15 nm, or any range of values between 10 and 1000 nm, e.g., 100-137 nm, in increments of 1.0 nm).
36. A composition comprising (i)(a) one or more amphiphilic copolymers, wherein each amphiphilic copolymer of said composition independently comprises the amphiphilic copolymer of any one of embodiments 1 to 33 or (i)(b) the nanocarrier of embodiment 34 or 35, and (ii) at least one biologically active substance combined with the one or more amphiphilic copolymers or the nanocarrier.
37. The composition of embodiment 36, wherein the at least one biologically active substance comprises a drug, an anti-carcinogenic compound, a nucleic acid, another small molecule, or any combination thereof.
38. The composition of embodiment 36 or 37, wherein the at least one biologically active substance comprises an anti-carcinogenic compound.
39. The composition of any one of embodiments 36 to 38, wherein the at least one biologically active substance comprises paclitaxel, docetaxel, carbazitaxel, ixabepilone, eribulin, topotecan, irinotecan, SN-38, doxorubicin, daunorubicin, idarubicin, epirubicin, etoposide, omacetaxine, or any combination thereof.
40. The composition of any one of embodiments 36 to 39, wherein the at least one biologically active substance comprises paclitaxel.
41. The composition of any one of embodiments 36 to 40, wherein the at least one biologically active substance comprises *let-7 mimic.* It should be noted that other nucleic acids may be used instead of or in combination with *let-7 mimic.* Other suitable nucleic acids include, but are not limited to, other miRNA mimics such as miR-34 mimic, anti-miRs, siRNAs, antisense oligonucleotides, and any combination thereof. Consequently, in all of the embodiments (and claims) herein, any other nucleic acid or combination of nucleic acids may be substituted for *let-7 mimic.*
42. The composition of any one of embodiments 36 to 41, wherein the at least one biologically active substance comprises a combination of paclitaxel and *let-7 mimic.*
43. The composition of embodiment 42, wherein the combination of paclitaxel and *let-7* mimic is present with the amphiphilic copolymer of embodiment 24 with the paclitaxel being associated with hydrophobic portions of the vitamin E units, and the *let-7 mimic* being associated with cationic portions of the diethylenetriamine groups.
44. The composition of any one of embodiments 36 to 43, wherein said composition comprises (1) a first amphiphilic copolymer of any one of embodiments 1 to 10, 15 to 16, 19 to 21, 25 to 26 and 30 to 31, and (2) a second amphiphilic copolymer of any one of embodiments 11 to 14 and 17 to 33, the at least one biologically active substance being incorporated within or along at least the second amphiphilic copolymer.
45. The composition of embodiment 44, wherein the first amphiphilic copolymer comprises the amphiphilic copolymer of embodiment 10; the second amphiphilic copolymer comprises the amphiphilic copolymer of embodiment 24; and the at least one biologically active substance comprises paclitaxel and *let-7 mimic* with the paclitaxel being associated with hydrophobic portions of the vitamin E units, and *let-7 mimic* being associated with cationic portions of the diethylenetriamine groups.
46. The composition of embodiment 44 or 45, wherein the first amphiphilic copolymer and the second amphiphilic copolymer are present at a weight ratio of from 1:1 to 16:1 (or any weight ratio between 1:1 and 16:1, e.g., 8:1, or any range of weight ratios between 1:1 and 16:1, e.g., from 6.5:1 to 12.8:1, in increments of 0.1).
47. The composition of any one of embodiments 44 to 46, wherein the first amphiphilic copolymer and the second amphiphilic copolymer are present at a weight ratio of 8:1.
48. The composition of any one of embodiments 44 to 47, further comprising a third amphiphilic copolymer comprising the amphiphilic copolymer of any one of embodiments 11 to 12, 19 to 23, 25 to 27 and 30 to 33, wherein R represents a crosslinking moiety.
49. The composition of embodiment 48, wherein each R represents thioctic acid.
50. The composition of embodiment 48 or 49, wherein the third amphiphilic copolymer comprises the amphiphilic copolymer of embodiment 12.
51. The composition of any one of embodiments 48 to 50, wherein a weight ratio of (i) a combined weight of the first amphiphilic copolymer and the third amphiphilic copolymer to (ii) the second amphiphilic copolymer is from 1:1 to 16:1 (or any weight ratio between 1:1 and 16:1, e.g., 8:1, or any range of weight ratios between 1:1 and 16:1, e.g., from 6.5:1 to 12.8:1, in increments of 0.1).
52. The composition of any one of embodiments 48 to 51, wherein a weight ratio of (i) a combined weight of the first amphiphilic copolymer and the third amphiphilic copolymer to (ii) the second amphiphilic copolymer is from 8:1.
53. The composition of any one of embodiments 36 to 43, wherein said composition comprises (1) a first amphiphilic copolymer of any one of embodiments 11 to 12, 19 to 23, 25 to 27 and 30 to 33, wherein R represents a crosslinking moiety, and (2) a second amphiphilic copolymer of any one of embodiments 11 to 14 and 17 to 33, wherein R represents a cationic moiety, the at least one biologically active substance being incorporated within or along at least the second amphiphilic copolymer.
54. The composition of embodiment 53, wherein each crosslinking moiety comprises thioctic acid.
55. The composition of embodiment 53 or 54, wherein each cationic moiety comprises diethylenetriamine.
56. The composition of any one of embodiments 53 to 55, wherein the first amphiphilic copolymer comprises the amphiphilic copolymer of embodiment 12; the second amphiphilic copolymer com-prises the amphiphilic copolymer of embodiment 24; and the at least one biologically active sub-stance comprises paclitaxel and let-7 mimic with the paclitaxel being associated with hydrophobic portions of the vitamin E units, and let-7 mimic being associated with cationic portions of the diethylenetriamine groups.
57. The composition of any one of embodiments 53 to 56, wherein the first amphiphilic copolymer and the second amphiphilic copolymer are present at a weight ratio of from 1:1 to 16:1 (or any weight ratio between 1:1 and 16: 1, e.g., 8:1, or any range of weight ratios between 1:1 and 16:1, e.g., from 6.5:1 to 12. 8:1, in increments of 0.1).
58. The composition of any one of embodiments 53 to 57, wherein the first amphiphilic copolymer and the second amphiphilic copolymer are present at a weight ratio of 8:1.
59. The composition of any one of embodiments 36 to 58, further comprising water.
60. The composition of any one of embodiments 36 to 59, further comprising one or more additional components selected from the group consisting of sodium phosphate, sodium chloride, glucose, HEPES, mannitol, and combinations thereof.
61. The composition of any one of embodiments 36 to 60, wherein said composition does not contain any organic solvents or surfactants.
62. The composition of any one of embodiments 36 to 61, wherein each amphiphilic copolymer is present in any amount of up to 20 mg/mL (or any amount between 0.001 mg/mL and 20 mg/ml., e.g., 10.2 mg/ml., or any range of amounts between between 0.001 mg/mL and 20 mg/mL, e.g., from 0.01 mg/mL to 8.002 mg/ml., in increments of 0.001). It should be noted that the each amphiphilic copolymer may be present in any amount depending upon a number of factors including, but not limited to, the size of the amphiphilic copolymer(s), the application (e.g., drug delivery), and the biologically active substance(s) used.
63. The composition of any one of embodiments 36 to 62, wherein each biologically active substance is present in any amount of up to 6.0 mM (or any amount between 0.001 mg/mL and 6.0 mg/mL, e.g., 4.2 mg/mL, or any range of amounts between between 0.001 mg/mL and 6.0 mg/mL, e.g., from 0.01 mg/mL to 3.458 mg/mL, in increments of 0.001). It should be noted that each biologically active substance may be present in any amount depending upon a number of factors including, but not limited
to, the size of the amphiphilic copolymer(s), the application (e.g., drug delivery), and the biologically active substance(s) used.

### II. Method of Making Amphiphilic Copolymers, Nanocarriers and Compositions Containing Amphiphilic Copolymers and/or Nanocarriers

The present invention further provides methods for making amphiphilic copolymers, nanocarriers and compositions containing amphiphilic copolymers and/or nanocarriers. Various embodiments of the present invention relating to methods for making amphiphilic copolymers, nanocarriers and compositions containing amphiphilic copolymers and/or nanocarriers are provided below.

### Methods of Making Amphiphilic Copolymers. Nanocarriers and Compositions Embodiments:

64. A method of making the amphiphilic copolymer of any one of embodiments 1 to 33, the nanocarrier of embodiment 34 or 35, or the composition of any one of embodiments 36 to 63, said method comprising: covalently bonding three or more vitamin E units to the polyethylene glycol.
65. The method of embodiment 64, wherein the covalently bonding step comprises: reacting a functionalized polyethylene glycol having a terminal amine end group and a terminal carboxyl end group with one or more divalent linkages; and reacting functional groups on the one or more divalent linkages with the three or more vitamin E units.
66. The method of embodiment 65, wherein the covalently bonding step further comprises: reacting one or more crosslinking moieties with either (i) the polyethylene glycol, (ii) the one or more divalent linkages, or both (i) and (ii).
67. The method of any one of embodiments 64 to 66, further comprising: combining the at least one biologically active substance with the amphiphilic copolymer or the nanocarrier.
68. The method of embodiment 67, wherein the at least one biologically active substance comprises paclitaxel, docetaxel, carbazitaxel, ixabepilone, eribulin, topotecan, irinotecan, SN-38, doxorubicin, daunorubicin, idarubicin, epirubicin, etoposide, omacetaxine, or any combination thereof.
69. The method of embodiment 67 or 68, wherein the at least one biologically active substance comprises paclitaxel.
70. The method of any one of embodiments 67 to 69, wherein the at least one biologically active substance comprises *let-7* mimic. As noted above, other nucleic acids may be used instead of or in combination with *let-7 mimic* with other suitable nucleic acids including, but are not limited to, other miRNA mimics such as miR-34 mimic, anti-miRs, siRNAs, antisense oligonucleotides, and any combination thereof. Consequently, in all of the embodiments (and claims) herein, any other nucleic acid or combination of nucleic acids may be substituted for *let-7 mimic.*
71. The method of any one of embodiments 67 to 70, wherein the at least one biologically active substance comprises a combination of paclitaxel and *let-7* mimic.
72. The method of embodiment 71, wherein the combination of paclitaxel and *let-7* mimic is combined with the amphiphilic copolymer of embodiment 24 so that the paclitaxel is associated with hydrophobic portions of the vitamin E units, and the *let-7* mimic is associated with cationic portions of the diethylenetriamine groups.
73. The method of any one of embodiments 64 to 72, said method comprising forming a composition comprising (1) a first amphiphilic copolymer of any one of embodiments 1 to 10, 15 to 16, 19 to 21, 25 to 26 and 30 to 31, and (2) a second amphiphilic copolymer of any one of embodiments 11 to 14 and 17 to 33, the at least one biologically active substance being incorporated within or along at least the second amphiphilic copolymer.
74. The method of embodiment 73, wherein the first amphiphilic copolymer comprises the amphiphilic copolymer of embodiment 10; the second amphiphilic copolymer comprises the amphiphilic copolymer of embodiment 24; and the at least one biologically active substance comprises paclitaxel and *let-* 7 mimic with the paclitaxel being associated with hydrophobic portions of the vitamin E units, and *let-* 7 mimic being associated with cationic portions of the diethylenetriamine groups.
75. The method of embodiment 73 or 74, wherein the first amphiphilic copolymer and the second amphiphilic copolymer are present at a weight ratio of from 1:1 to 16:1 (or any weight ratio between 1:1 and 16: 1, e.g., 8:1, or any range of weight ratios between 1:1 and 16:1, e.g., from 6.5:1 to 12.8:1, in increments of 0.1).
76. The method of any one of embodiments 73 to 75, wherein the first amphiphilic copolymer and the second amphiphilic copolymer are present at a weight ratio of 8:1.
77. The method of any one of embodiments 73 to 76, further comprising forming the composition with a third amphiphilic copolymer comprising the amphiphilic copolymer of any one of embodiments 11 to 12, 19 to 23, 25 to 27 and 30 to 33, wherein R represents a crosslinking moiety.
78. The method of embodiment 77, wherein each R represents thioctic acid.
79. The method of embodiment 77 or 78, wherein the third amphiphilic copolymer comprises the amphiphilic copolymer of embodiment 12.
80. The method of any one of embodiments 77 to 79, wherein a weight ratio of (i) a combined weight of the first amphiphilic copolymer and the third amphiphilic copolymer to (ii) the second amphiphilic copolymer is from 1:1 to 16:1 (or any weight ratio between 1: 1 and 16: 1, e.g., 8:1, or any range of weight ratios between 1:1 and 16:1, e.g., from 6.5:1 to 12.8:1, in increments of 0.1).
81. The method of any one of embodiments 77 to 80, wherein a weight ratio of (i) a combined weight of the first amphiphilic copolymer and the third amphiphilic copolymer to (ii) the second amphiphilic copolymer is from 8:1.
82. The method of any one of embodiments 64 to 72, said method comprising forming a composition comprising (1) a first amphiphilic copolymer of any one of embodiments 11 to 12, 19 to 23, 25 to 27 and 30 to 33, wherein R represents a crosslinking moiety, and (2) a second amphiphilic copolymer of any one of embodiments 11 to 14 and 17 to 33, wherein R represents a cationic moiety, the at least one biologically active substance being incorporated within or along at least the second amphiphilic copolymer.
83. The method of embodiment 82, wherein each crosslinking moiety comprises thioctic acid.
84. The method of embodiment 82 or 83, wherein each cationic moiety comprises diethylenetriamine.
85. The method of any one of embodiments 82 to 84, wherein the first amphiphilic copolymer comprises the amphiphilic copolymer of embodiment 12; the second amphiphilic copolymer comprises the amphiphilic copolymer of embodiment 24; and the at least one biologically active substance comprises paclitaxel and *let- 7 mimic* with the paclitaxel being associated with hydrophobic portions of the vitamin E units, and *let- 7 mimic* being associated with cationic portions of the diethylenetriamine groups.
86. The method of any one of embodiments 82 to 85, wherein the first amphiphilic copolymer and the second amphiphilic copolymer are present at a weight ratio of from 1:1 to 16:1 (or any weight ratio between 1:1 and 16:1, e.g., 8:1, or any range of weight ratios between 1:1 and 16:1, e.g., from 6.5:1 to 12.8:1, in increments of 0.1).
87. The method of any one of embodiments 82 to 86, wherein the first amphiphilic copolymer and the second amphiphilic copolymer are present at a weight ratio of about 8:1.
88. The method of any one of embodiments 64 to 87, further comprising: incorporating the at least one biologically active substance and each amphiphilic copolymer or nanocarrier into an aqueous solution.

### III Methods of Using the Reaction Product of PEG and Vitamin E

The present invention is even further directed to methods of using the amphiphilic copolymers, nanocarriers and compositions containing amphiphilic copolymers and/or nanocarriers. Various embodiments of the present invention relating to methods for using amphiphilic popolymers, nanocarriers and compositions containing amphiphilic copolymers and/or nanocarriers are provided below.

The present invention is further illustrated by the following examples.

### EXAMPLES

### Materials Used in Examples

Monomethylterminated poly(ethylene glycol) monoamine, HCl salt (CH₃O-PEG-NH₂·HCl) with number average molecular weight (Mn) of 5000 g/mol and polydispersity index (PDI) 1.04 (GPC) was purchased from Jenkem Technology (Beijing, China) and used without further purification.

N_{α}-Fmoc-N_{ε}-Boc-L-lysine (Fmoc- Lys(Boc)-OH) (i. e., N_{α}-fluorenylmethyloxy- carbonyl-N_{ε}-butyloxycarbonyl-L-lysine), and N_{α},N_{ε}-Fmoc-L-lysine (Fmoc-Lys(Fmoc)-OH) were purchased from P3Biosystems (Shelbyville, KY).

N,N'-Diisopropylcarbodiimide (DIC), N-hydroxysuccinimide (NHS), ethyldiisopropylamine (DIEA), piperidine and trifluoroacetic acid (TFA) and triphosgene were obtained from Alfa Aesar (Ward Hill, MA).

Vitamin E was purchased from Sigma (St. Louis, MO). 1-Hydroxybenzotriazole hydrate (HOBt) and L-aspartic acid beta-benzyl ester were purchased from AK Scientific (Union City, CA).

β-Benzyl-L-aspartate N-carboxy anhydride (BLA-NCA) was carried out by the Fuchs-Farthing method using 1 equivalent of beta-benzyl-L-aspartate and 1.2 equivalents of triphosgene in tetrahydrofuran (THF).

Paclitaxel was purchased from LC Laboratories (Woburn, MA).

1,10-Dioctadecyl-3,3,30,30-tetramethylindodicarbocyanine perchlorate (DiD) was purchased from Invitrogen (Grand Island, NY).

3,3'-Dioctadecyloxacarbocyanine, perchlorate (DiO) and 1,1'-dioctadecyl-3,3,3',3'-tetramethylindocarbocyanine perchlorate (DiI) were obtained from Biotium (Hayward, CA).

All solvents were of ACS grade or higher and were purchased from Alfa Aesar.

Lipofectamine 2000, LysoTraker Deep Red and Oregon green-labeled paclitaxel were purchased from Life Technologies (Grand Island, NY).

*Let-7b-5p* mimic and the Cy3 labeled*-let-7b-5p* mimic were purchased from Bioneer (Alameda, CA).

### Example 1 - Preparation and Use of PEG:VE:TA Copolymer

In this example of the present invention, a novel copolymer comprised of polyethylene glycol 5000 (PEG_{5K}), Vitamin E (VE) and thioctic acid (TA) with a molar ratio of PEG:VE:TA at 1:4:4 was prepared. The resulting PEG_{5K}-VE₄-TA₄ copolymer self-assembled into micelles in aqueous solution, which could be readily crosslinked by a catalytic amount of dithiothreitol to form polydisulfide crosslinks within the hydrophobic region of the micelles. The crosslinked PEG_{5K}-VE₄-TA₄ micelles had an average particle size of 44 nm, a neutral surface charge and a maximum loading for paclitaxel of 4.3 mM, nearly identical to the characteristics of the non-crosslinked micelles. The crosslinking of the PEG_{5K}-VE₄-TA₄ micelles not only enhanced thermodynamic and kinetic stability, but also deterred the release of paclitaxel from the micelles. The polydisulfide crosslinks were responsive to the intracellular level of glutathione, which caused rapid disassembly of the micelles and accelerated drug release. Intravenous administration of paclitaxel-loaded crosslinked PEG_{5K}-VE₄-TA₄ micelles yielded approximately 3-fold and 5-fold higher plasma concentration than that of the non-crosslinked counterparts and Taxol®, respectively. In mice bearing human ovarian SKOV-3 tumors, *in vivo* imaging study revealed that intravenous injection of DiD-labeled crosslinked micelles resulted in 3-fold higher near infrared fluorescence intensity in SKOV-3 tumors than that of the non-crosslinked micelles at 48 h. Moreover, paclitaxel-loaded crosslinked micelles exerted significantly more potent tumor growth inhibition than the non-crosslinked counterparts and Taxol®. The crosslinked PEG_{5K}-VE₄-TA₄ nanocarrier system was shown to represent a promising platform for the delivery of hydrophobic anticancer agents.

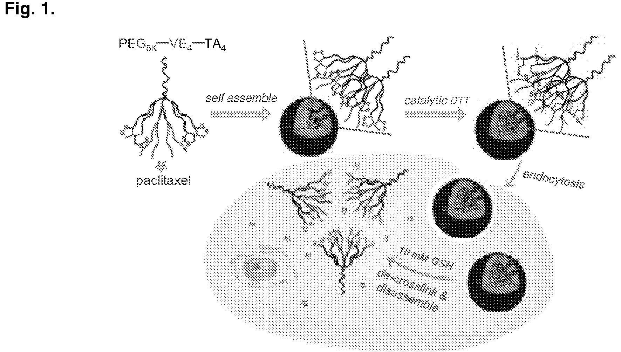

As discussed further below, nanocarriers were synthesized and evaluated. The reduction-responsive nanocarrier system was based on TA-grafted PEG_{5K}-VE₄ copolymer (see, FIG. 1). It could self assemble into micelles in aqueous solution with a high loading capacity for paclitaxel. By forming polydisulfide crosslinks, thermodynamic stability of the crosslinked PEG_{5K}-VE₄-TA₄ micelles was markedly improved as reflected by a 10-fold decrease in the CMC (0.19 mg/L) than that of the noncrosslinked counterparts (2.26 mg/L). Moreover, the crosslinked PEG_{5K}-VE₄-TA₄ micelles remained intact in the presence of micelle-disrupting agents, signifying enhanced kinetic stability. While the release of paclitaxel from the crosslinked micelles was much deterred in the non-reducing environment, the crosslinked PEG_{5K}-VE₄-TA₄ micelles were responsive to GSH at the intracellular level, which accelerated drug release due to rapid de-crosslinking and disassembly of the micelles. Intravenous administration of paclitaxel formulated in the crosslinked PEG_{5K}-VE₄-TA₄ micelles resulted in about 3-fold and 5-fold higher plasma concentration than that of the non-crosslinked counterparts and Taxol®, respectively, leading to superior anticancer efficacy in mice bearing human ovarian cancer SKOV-3 xenografts.

### Synthesis of Copolymers

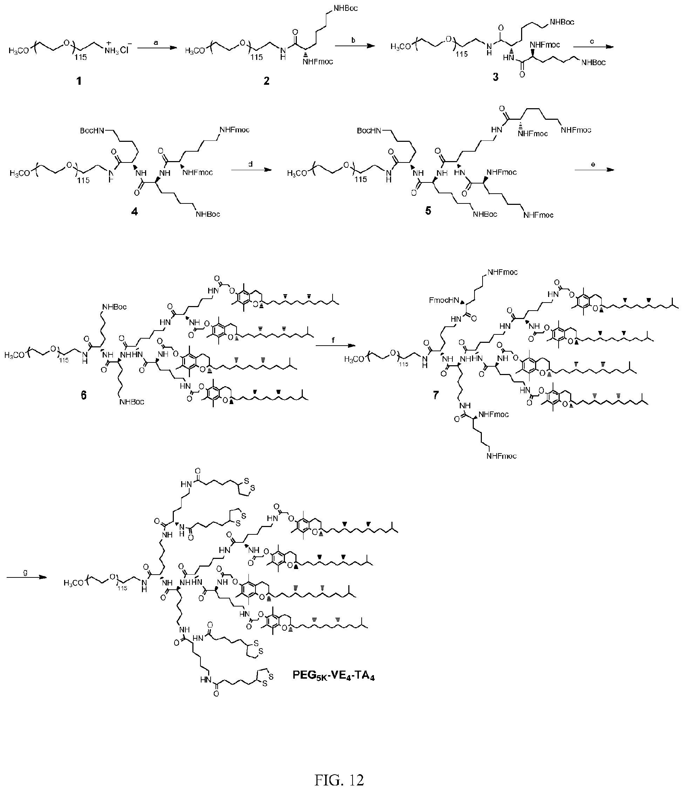

The PEG_{5K}-VE₄-TA₄ copolymer was synthesized via stepwise peptide condensation reactions from MeO-PEG-NH₂·HCl. PEG_{5K}-NH₂ (see, component **1** shown in FIG. **12**) (2 g, 0.4 mmol) was coupled by Fmoc-Lys(Boc)-OH (375 mg, 0.8 mmol) in the presence of DIC (150 µL, 0.96 mmol), HOBt (164 mg, 0.96 mmol) and DIEA (350 µL, 2 mmol) in DMF (50 mL) for 3 h at room temperature. The reaction mixture was poured into diethyl ether (200 mL) until the completion of the coupling reaction indicated by the Kaiser test. The precipitates were collected after centrifugation, washed by fresh ether (3×150 mL) and dried under vacuum to yield PEGylated compound **2** shown in FIG. **12**.

Fmoc groups were de-protected by the treatment with piperidine in DMF (v/v 1:4, 100 mL). The PEGylated compound were precipitated and washed with cold ether (200 mL). One more coupling of Fmoc-Lys(Boc)-OH and two more coupling of Fmoc-Lys(Fmoc)-OH were carried out step by step to furnish PEGylated compound **5** shown in FIG. **12**. After the removal of Fmoc groups by piperidine in DMF, α-tocopheryloxyacetic acid synthesized according to a known method in the art (i.e., the method disclosed in Lawson KA, Anderson K, Menchaca M, Atkinson J, Sun L, Knight V, et al. "Novel Vitamin E analogue decreases syngeneic mouse mammary tumor burden and reduces lung metastasis", Mol Cancer Ther 2003; 2:437-44, the subject matter of which is hereby incorporated by reference in its entirety) was conjugated to the amino terminals of the lysine residues to afford PEGylated compound **6** shown in FIG. **12**.

The Boc groups of PEGylated compound **6** were removed by TFA in dichloromethane (v/v 1:3, 20 mL) for 30 min. The mixture was precipitated in cold ether and collected following centrifugation. Two coupling of Fmoc-Lys(Fmoc)-OH and one coupling of thioctic acid to the side amino groups of the PEGylated compound were further carried out according to the typical conjugation procedure. The PEG_{5K}-VE₄-TA₄ copolymer was recovered from the mixture by repeated precipitation from DMF into diethyl ether. Finally, the copolymer was further purified by dialysis against deionized water for 2 days and lyophilized to obtain an off-white solid. 1H NMR spectra of the copolymers were collected at 20°C on a 600 MHz Agilent Inova Spectrometer (Santa Clara, CA) using CDCl₃ as a solvent.

In summary, the reaction scheme shown in FIG. **12** utilized to following reagents and reaction conditions in steps (a) to (g):
(a) Fmoc-Lys(Bos)-OH, DIC, HOBt, DIEA, DMF, r.t, 6 h.
(b)(i) 20% Piperidine in DMF, r.t, 2 h.; and (ii) Fmoc-Lys(Bos)-OH, DIC, HOBt, DIEA, DMF, r.t, 6 h.
(c)(i) 20% Piperidine in DMF, r.t, 2 h.; and (ii) Fmoc-Lys(Fmoc)-OH, DIC, HOBt, DIEA, DMF, r.t, 6 h.
(d)(i) 20% Piperidine in DMF, r.t, 2 h.; and (ii) Fmoc-Lys(Fmoc)-OH, DIC, HOBt, DIEA, DMF, r.t, 6 h.
(e)(i) 20% Piperidine in DMF, r.t, 2 h.; and (ii) α-tocopheryloxyacetic acid, DIC, NHS, DIEA, DMF, r.t, overnight.
(f)(i) 50% TFA in CHCl₃, r.t, 1 h.; and (ii) Fmoc-Lys(Fmoc)-OH, DIC, HOBt, DIEA, DMF, r.t, 6 h.
(g)(i) 20% Piperidine in DMF, r.t, 2 h.; and (ii) lipoic acid, DIC, NHS, DMF, DMF, r.t, overnight.

### Preparation of the PEG_{5K}-VE₄-TA₄ Micelles

### Preparation of the Blank Micelles

The blank micelles were prepared by dissolving 90 mg PEG_{5K}-VE₄-TA₄ copolymer in 1 mL phosphate buffered saline (PBS) following 20 min sonication. The micelle solution was divided equally into two portions, one of which remained unmodified to be the blank non-crosslinked micelles (B-NCMs). To the second portion, nitrogen gas (N₂) was bubbled for 10 min, and 10 µL of DTT (30 mg/mL in PBS) was then added to the micelle solution under a N₂ atmosphere. The solution was shaken at 4°C for 24 h to form the blank crosslinked micelles (B-CMs). The UV absorbance of the micelle solution was measured at 330 nm to confirm the formation of the crosslinks among the TA moieties.

### Preparation of Paclitaxel-Loaded Micelles and the NIR Dye-Loaded Micelles

A solvent evaporation method was employed to prepare the drug-loaded micelles similar to the procedure reported in Katragadda U, Fan W, Wang Y, Teng Q, Tan C. "Combined delivery of paclitaxel and tanespimycin via micellar nanocarriers: pharmacokinetics, efficacy and metabolomic analysis", PLoS One 2013;8:e58619, the subject matter of which is hereby incorporated by reference in its entirety. Briefly, the varying amount of paclitaxel and 90 mg PEG_{5K}-VE₄-TA₄ were dissolved in 2 mL chloroform in a 25-mL round-bottom flask, which was rotor evaporated to dryness at room temperature to form a homogenous thin drug-copolymer film. The resulting thin film was further dried overnight under vacuum to remove any residual solvent. Paclitaxel-loaded non-crosslinked micelles (P-NCMs) were formed by hydrating the film in 1 mL PBS followed by 20 min sonication. To prepare the crosslinked micelles, N₂ was bubbled into 500 µL P-NCMs for 10 min, and 10 µL DTT (30 mg/mL in PBS) was then added to the micelle solution under a N₂ atmosphere. The solution was shaken at 4°C for 24 h to form paclitaxel-loaded crosslinked micelles (P-CMs).

The loading concentration of paclitaxel in the micelles, defined as the amount of the drugs in the resulting micellar solution per unit volume of PBS, was quantified by HPLC as described below. The encapsulation efficiency of paclitaxel was calculated as the percentage of the incorporated vs. the input paclitaxel.

DiD and paclitaxel were co-loaded into the non-crosslinked (DP-NCMs) and crosslinked micelles (DP-CMs) using the same method as described above with the final DiD concentration as 0.1 mg/mL. DiO and DiI, a lipophilic florescence energy transfer (FRET) pair, were co-loaded into the crosslinked micelles using the same method with a final concentration of 0.25 mg/mL for each dye. All micelle formulations were filtered with 0.22 µM syringe filter for sterilization.

### Characterization of the PEG_{5K}-VE₄-TA₄ Micelles

### Size and Zeta Potential

The hydrodynamic diameter and zeta potential of the micelles were recorded by dynamic light scattering (DLS) using a Nano Zetasizer ZS (Malvern Instruments, UK) equipped with He-Ne laser (4 mW, 633 nm) light source and 90° angle scattered light collection configuration.

### Transmission Electron Microscopy (TEM)

The morphology of the micelles was observed at room temperature using a Tecnai G2 Spirit TEM (FEI, Hillsboro, OR). A drop of the micellar solution was deposited on a carbon-coated copper grid, dried at room temperature and negatively stained with 1% phosphotungstic acid before TEM visualization.

### Critical Micelle Concentration (CMC)

The CMC was determined by fluorescence spectra using pyrene as a fluorescent probe. Pyrene solution (20 µL, 0.5 mM in ethanol) was added into the glass tubes, and the solvent was evaporated at room temperature. B-NCMs or B-CMs solution was diluted in deionized water to the copolymer concentration ranging from 0.1-50 mg/L, which was added to the pyrene-containing glass tubes and gently shaken for 24 h to reach equilibrium before measurement. The fluorescence spectra of pyrene were recorded by a fluorescence spectrophotometer (Perkin Elmer, USA). The excitation wavelength at 331 nm and the emission fluorescence intensity at 373 nm and 393 nm values were used. The CMC was estimated from the threshold of the copolymer concentration, where the intensity ratio 1393/1373 begins to increase markedly.

### Stability Studies of the PEG_{5K}-VE₄-TA₄ Micelles

The storage stability of the PEG_{5K}-VE₄-TA₄ micelles in PBS was assessed by monitoring paclitaxel concentration and the micelle size following incubation at 4°C for 4 weeks. To study the stability of the micelles in sodium dodecyl sulfate (SDS), P-NCMs or P-CMs were incubated in PBS containing 2.5 mg/mL SDS at room temperature for 90 min. To evaluate the stability of the micelles in serum, the micelles were incubated in PBS containing 50% fetal bovine serum (FBS) at 37°C for 24 h. The stability of P-CMs in PBS containing 2 µM or 10 mM GSH was studied at 37°C for 12 h. The size of the micelles was monitored by DLS. All samples were in triplicates.

### In vitro Release Study of Paclitaxel from the PEG_{5K}-VE₄-TA₄ Micelles

The release of paclitaxel from the PEG_{5K}-VE₄-TA₄ micelles was studied by a dialysis method. Paclitaxel-loaded micelles in PBS was inserted into a Slid-A-Lyzer dialysis cassette (Thermo Scientific, Rockford, IL) with a 20 KDa MWCO. The cassette was submerged into 50 mL of PBS as the dialysis media at 37°C and swirled at 100 rpm. At predetermined time intervals, the sample was withdrawn from the cassette, the paclitaxel concentration was quantified by HPLC, and the dialysis media was refreshed to maintain the sink condition. To further investigate the release profile of the crosslinked micelles in response to reduction, the cassettes were also dialyzed against 50 mL PBS containing 2 µM or 10 mM GSH to mimic the extracellular and intracellular GSH level, respectively.

### Cell Culture

Human ovarian cancer SKOV-3 cells (American Type Culture Collection, Manassas, VA) were grown in DMEM medium (Invitrogen, Carlsbad, CA) with 2 mM L-glutamine, which was supplemented with 10% (v/v) fetal bovine serum, 100 units/mL penicillin G and 10 µg/mL streptomycin. The cells were maintained at 37°C with 5% CO₂ in a humidified incubator.

### Cellular Uptake of the Crosslinked PEG_{5K}-VE₄-TA₄ Micelles

To study the uptake of the crosslinked micelles, SKOV-3 cells were treated with DiI/DiO-loaded crosslinked micelles for 4, 6, 12 and 24 h. After removing the culture medium, cells were washed 3 times with cold PBS, and then trypsinized and re-suspended in PBS before being subjected to the flow cytometry analysis. Cell-associated fluorescence was analyzed using a BD Accuri C6 Flow Cytometer System (San Jose, CA). The fluorescence signals were acquired at the excitation wavelength of 488 nm. The spectral filters of 530 ± 15 nm and 585 ± 20 nm was used to detect DiO and DiI, respectively. All intensities were obtained with the same gain and offset. Data were analyzed using the FlowJo 9.3.1 software (Tree Star, Inc., Ashland, OR) and expressed as the geometric mean of the entire cell population.

### Cytotoxicity Assay

SKOV-3 cells were seeded at a density of 6,000 cells/well in 96-well plate, and treated in triplicates with various concentrations of blank micelles (10-3-10 mg/mL) or paclitaxel (1-100 nM) either in the free form, non-crosslink or cross-linked micelles for 72 h. Cells were then fixed with 1% glutaraldehyde, stained with 0.1% crystal violet, and dissolved in 10% acetic acid. The absorbance was quantified at 595 nm on FLUOstar Omega plate reader (Cary, NC). The relative cell viability was calculated as the percentage of absorbance of the treated vs. the untreated wells. Results are reported as the means for each triplicate samples.

### Tumor Xenograft Murine Model

All animal procedures were performed in strict accordance with the Guide for the Care and Use of Laboratory Animals of the National Institutes of Health. The protocol was approved by the Institutional Animal Care and Use Committee at Mercer University (Approval Number: A1105007). SKOV-3 cells (2 × 106 in 0.1 mL matrigel/DMEM mixture) were subcutaneously implanted in each flank of 5-6 week-old female athymic nude mice (*nu*/*nu,* Charles River, Wilmington.)

### Pharmacokinetic Study

When the tumor volumes reached 100-300 mm³, the mice were randomized into three treatment groups (n=3). Each group was i.v. administrated with Taxol®, P-NCMs and P-CMs at an identical dose of 5 mg/kg paclitaxel. The loading concentration of paclitaxel in both P-NCMs and P-CMs was 0.5 mM. At pre-determined time point, namely 5, 15, 30, 45, 60 and 90 min post administration, the blood samples were collected via retro-orbital bleeding. The concentration of paclitaxel in plasma was quantified by HPLC as described below. Non-compartmental analysis was performed to obtain the pharmacokinetic parameters including the elimination half-life (t_{1/2,e}), the total clearance (CL_{T}), and the apparent volume of distribution (V_{d,ss}) using the WinNonlin software (version 5.1, Pharsight, Sunnyvale, CA).

### HPLC Methodology

The Waters HPLC system (Milford, MA) consisted of a 2795 pump with an autosampler, a 996 photodiode array detector and a Phenomenex (Torrance, CA) C8 column (5 µm, 4.6 mm × 150 mm). α-Naphthoflavone was used as an internal standard. The detection wavelengths for paclitaxel and α-naphthoflavone were 227 nm and 281 nm, respectively. An isocratic mobile phase of 28% (v/v) sodium phosphate buffer (25 mM, pH 3.0) with 10 mM triethylamine and 72% (v/v) methanol was used at a flow rate of 1.5 mL/min. The plasma samples were extracted by ethyl acetate, reduced to dryness under vacuum, and reconstituted with the HPLC mobile phase prior to HPLC analysis.

### In vivo NIR Imaging and Tissue Distribution

DiD, a lipophilic NIR dye (excitation: 644 nm, emission: 665 nm), was used to label paclitaxel-loaded PEG_{5K}-VE₄-TA₄ micelles. DiD and paclitaxel were co-loaded into the micelles to form DP-NCMs and DP-CMs with a final paclitaxel concentration of 0.5 mM as described in Section 1.3.2. When the tumor volume reached 100-300 mm³, the mice were randomized into two treatment groups (n=3), and were i.v. administrated with DP-NCMs or DP-CMs at a dose of 1.6 mg/kg DiD. At 0.083, 4, 8, 24 and 48 h post administration, the mice were anaesthetized by isoflurane and the whole-body images of the mice were scanned by Odyssey imaging system (LI-COR, Lincoln, NE). At 24 and 48 h, the mice were euthanized and the major organs including heart, liver, spleen, lung and kidney as well as the tumor tissues were excised and rinsed with PBS. The NIR signals were scanned and analyzed by Odyssey imaging system. The NIR intensity was quantified using the Odyssey Application Software (Version 3.021).

### Antitumor Efficacy Study

When the tumor volumes reached 50-100 mm³, the mice were randomized into five treatment groups (n=5): (A) the untreated group; (B) the B-CMs group; (C) the Taxol® group; (D) the P-NCMs group; and (E) the P-CMs group. For Group B, the mice were i.v. administrated with an equal amount of the PEG_{5K}-VE₄-TA₄ copolymer as Groups D and E. For Groups C, D, and E, the mice were i.v. administered with different paclitaxel formulations at 5 mg/kg twice-weekly for 3 weeks. The final concentration of paclitaxel in P-NCMs and P-CMs was 0.5 mM. The tumor size was measured using a caliper twice-weekly, and the tumor volume was calculated as 1/2 × length × width². To monitor the overall toxicity, the body weight of all mice from the different groups was measured twice-weekly. The tumors were excised and divided for immediate formalin fixation and flash freezing (-80 °C) for the histology/immunohistochemistry analysis and Western blotting.

### Histology and Immunohistochemistry

Formalin-fixed and paraffin-embedded tumor specimens of 3- to 4-µm thickness were sectioned and stained with hematoxylin and eosin (H&E) and the proliferating cell nuclear antigen (PCNA) antibody following the standard protocols.

### Western Blot Analysis

Tumor tissue samples (approximately 20-25 mg per sample) were homogenized and lysed using the RIPA buffer supplemented with the protease and phosphatase inhibitors. Tumor lysates containing 35 µg of total proteins were resolved onto 10% SDS-PAGE gels, and transferred onto the nitrocellulose membranes. Blots were probed with the antibodies for PCNA, phosphorylated Akt (p-Akt) and total Akt, all of which were from Cell Signaling (Danvers, MA). Anti-β-actin antibody (Santa Cruz Biotechnology, Inc. (Dallas, TX) was used as a loading control.

### Statistical Analysis

All data are presented as the mean ± standard deviation (SD). Data from the different groups were compared using the Student's *t*-test. A *p*-value of less than 0.05 was considered statistically significant.

### Results and Discussion

### Synthesis and Characterization of PEG_{5K}-VE₄-TA₄ Copolymer

The synthesis of PEG_{5K}-VE₄-TA₄ is shown in FIG. 12 as Scheme S1. The completed reaction for each step was confirmed by the Kaiser test and thin layer chromatography. The structures of the PEG_{5K}-VE₄-TA₄ copolymer and intermediates **5** and **6** were confirmed by 1H-NMR.

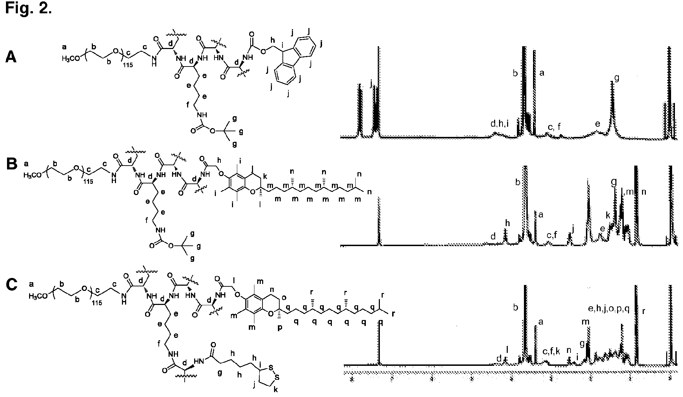

As shown in FIG. **2A**, the peaks for the aromatic hydrogen of the Fmoc group in compound **5** was observed at 7.2-7.8 ppm, with the characteristic peaks of the PEG chain (-CH2CH2O-) at 3.7 ppm and Boc hydrogen appearing at 1.3 ppm. The disappearance of the aromatic peaks and the presence of peaks for the alkyl hydrogen at 0.8-1.5 ppm indicate the complete de-protection of the Fmoc groups and the successful conjugation of VE in intermediate **6** (see, FIG. **2B**). The thioctic acid (TA) structure could be observed from the peaks at 1.5-3.2 ppm in FIG. **2C**. Moreover, ¹H NMR showed that the alkyl peaks (0.8-1.4 ppm) and the phenymethyl peak (2.1 ppm) were not changed, indicating that the VE component remained intact during the Boc group de-protection by TFA. The average number of VE substituents per PEG chain could be determined by comparing the peak intensity of the terminal methyl group in VE (0.8 ppm) and the PEG methylene protons (3.70 ppm) in FIG. **2B**. The average number of TA substituents per copolymer molecule could be also calculated by comparing the peak intensity at 2.2 ppm (a-methylene neighboring to the carbonyl group of TA) and 4.2 ppm (a-methylene neighboring to the carbonyl group of tocopheryloxyacetic acid) in FIG. **2C**. The numbers of VE and TA units per PEG chain were 4.12 and 3.91, respectively, which were in good agreement with the molecular formula of the target copolymer.

### Preparation and Characterization of the PEG_{5K}-VE₄-TA₄ Micelles

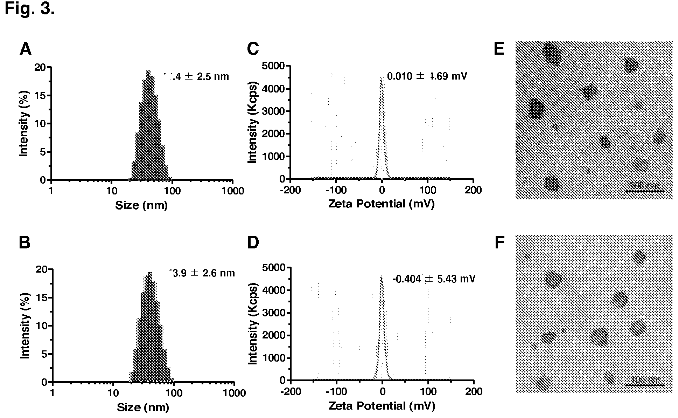

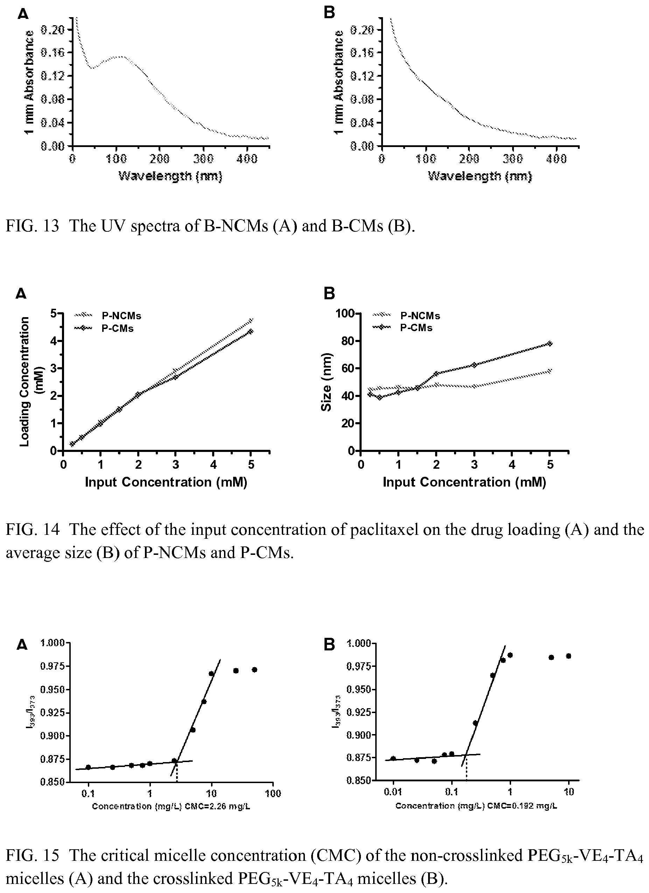

The crosslinking of the micelles was achieved by introducing DTT to the TA units in the micelles with a molar ratio of DTT:TA at 1:10 for 24 h. The UV spectra of the blank PEG_{5K}-VE₄-TA₄ micelles in water showed that the absorbance of the disulfide-containing thioctic ring at 330 nm, which completely disappeared after crosslinking (see, FIGS. **13A-****13B**), indicating the breakage of the disulfide bond within the thioctic ring and the formation of linear polydisulfide bonds among adjacent TA units. Paclitaxel-loaded PEG_{5K}-VE₄-TA₄ micelles were further characterized with respect to their particle size and zeta potential (see Table 1 below and FIGS. **3A-3D**).

**Table 1. Characteristics of P-NCMs and P-CMs.**

| Micelles | Size (nm) | Zeta Potential (mV) | PDI | CMC (mg/L) | Maximum Loading (mM) |
|---|---|---|---|---|---|
| P-NCMs | 45.4±2.5 | 0.010±4.69 | 0.119 | 2.26 | 4.72 |
| P-CMs | 43.9±2.6 | -0.404±5.43 | 0.144 | 0.192 | 4.33 |

Results show in Table 1 representative data obtained from at least three independent experiments and are reported as the mean ± SD (n=3).

The average diameters of the crosslinked micelles (P-CMs) and their non-crosslinked counterparts (P-NCMs) were 43.9 ± 2.6 nm and 45.4 ± 2.5 nm, respectively. Their nearly identical sizes indicate that the cross-linkages among the TA moieties occur within the micelles, which has little influence on the hydrodynamic diameter of the micelles. The polydispersity indexes (PDI) of both formulations were less than 0.15, pointing to the narrow size distribution of the micelles and minimal inter-micellar aggregation during the crosslinking process. As expected, both micelle types had a close-to-neutral surface potential because of the uncharged PEG_{5K}-VE₄-TA₄ copolymer. The spherical morphology and the size distribution of P-CMs and P-NCMs were confirmed by TEM (see, FIGS. **3E** and **3F**).

To investigate the encapsulation capacity of the micelles for paclitaxel, PEG_{5K}-VE₄-TA₄ copolymer at 10 mg/mL was used to encapsulate increasing input concentrations of paclitaxel, and the loading concentration and size distribution of the micelles were monitored. As shown in Table 1 and FIG. **14A**, the maximum loading of paclitaxel in P-NCMs and P-CMs was 4.72 mM and 4.33 mM, respectively, with the encapsulation efficiency of 94.4% and 86.8%. There was no significant size alteration of P-NCMs as the input concentration of paclitaxel was increased, while the size of P-CMs began to increase once the input concentration exceeded 2 mM (FIG. **14B**).

The CMC of the crosslinked PEG_{5K}-VE₄-TA₄ micelles was 0.192 mg/L (FIGS. **15A-****15B**), which was over 10-fold lower than that of the non-crosslinked counterparts (i.e., 2.26 mg/L). The pronounced decrease in the CMC of the crosslinked micelles provides strong evidence that the formation of polydisulfide crosslinks among adjacent PEG_{5K}-VE₄-TA₄ copolymers within the hydrophobic region improves thermodynamic stability of the crosslinked micelles.

### In vitro Stability of PEG_{5K}-VE₄-TA₄ Micelles

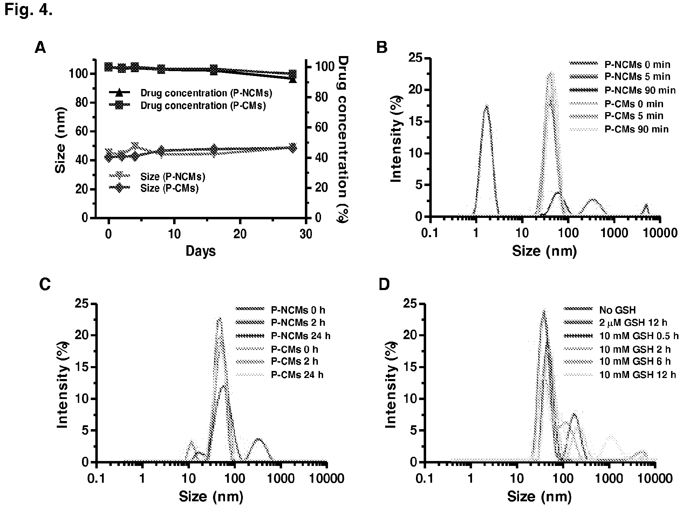

The storage stability of P-NCMs and P-CMs in PBS was evaluated by monitoring the size and paclitaxel concentration. Both micelle types displayed excellent stability in PBS at 4 °C for at least 4 weeks, reflected by little alteration in the particle size and less than 10% decrease in paclitaxel concentration in the micelle solution (see, FIG. **4A**). To investigate kinetic stability of paclitaxel-loaded PEG_{5K}-VE₄-TA₄ micelles, the size of paclitaxel-loaded PEG_{5K}-VE₄-TA₄ micelles was monitored in the presence of 2.5 mg/mL SDS, a strong surfactant that destabilizes supramolecular assemblies. SDS can form micelles itself in aqueous buffer with a particle size of less than 3 nm, which could aggregate with the PEG_{5K}-VE₄-TA₄ unimers in the micellar solution, thus breaking the exchange equilibrium between the unimers and the PEG_{5K}-VE₄-TA₄ micelles and eventually causing the disintegration of micelle assemblies. As shown in FIG. **4B**, in SDS-containing PBS the noncrosslinked micelles began to lose their initial size as early as 5 min, and almost completely disintegrated after 90-min incubation with the appearance of multiple particle peaks at different sizes. In contrast, the crosslinked micelles maintained their original size for at least 90 min despite the presence of SDS.

When the micelles are administered intravenously, they first encounter the plasma proteins and the blood cells before reaching the target tissues, which may cause the destabilization of micelles and premature release of the payload. It is therefore desirable for the drug-loaded micelles to sustain the destabilizing environment in the bloodstream and remain associated during the circulation. To evaluate the stability of the micelles in serum, the particle size of micelles in 50% serum was monitored. As shown in FIG. **4C**, the size of P-CMs was unaffected following 24 h-incubation in serum, while P-NCMs started to lose structural integrity within 2 h and formed larger aggregates at 24 h. Taken together, the above findings demonstrate that the crosslinked PEG_{5K}-VE₄-TA₄ micelles possess improved kinetic stability. The polydisulfide crosslinks formed within the hydrophobic region of the micelles covalently link the PEG_{5K}-VE₄-TA₄ copolymers and resist their dissociation from the micelle assemblies in the presence of micelle-disrupting agents.

It is known that the intracellular GSH level (1-10 mM) is much higher than the extracellular level (2-10 µM). To study the reduction sensitivity of the crosslinked PEG_{5K}-VE₄-TA₄ micelles, the change in the micelle size triggered by GSH at different time intervals was monitored (see, FIG. **4D**). The particle size of P-CMs remained unchanged in PBS containing 2 µM glutathione during 12-h incubation, indicating excellent stability of the crosslinked PEG_{5K}-VE₄-TA₄ micelles in the non-reducing environment found in the bloodstream and tissue fluids. In contrast, multimodal peaks of larger aggregates appeared when the crosslinked micelles were incubated with 10 mM GSH for only 0.5 h. These results indicate that the polydisulfide crosslinks within the P-CMs could be rapidly reduced and decrosslinked by the intracellular level of GSH, leading to the disassembly of the micelles.

### In vitro Release Study

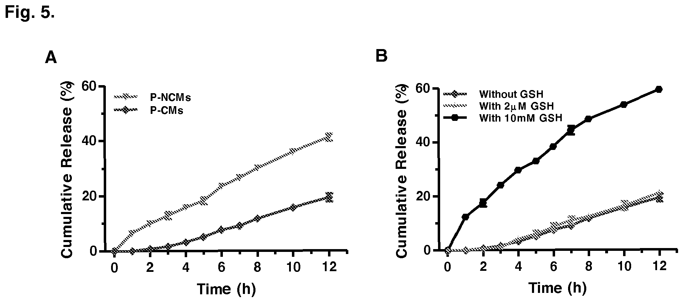

To evaluate the potential of the PEG_{5K}-VE₄-TA₄ micelles as drug carriers, the release kinetics of paclitaxel from the crosslinked PEG_{5K}-VE₄-TA₄ micelles were studied in comparison to the non-crosslinked counterparts. As shown in FIG. **5A**, the release of paclitaxel from P-CMs was slow at the early time points, and there was less than 5% of drug release at 4 h, while P-NCMs lost 15.8% of the drug content over the same period. About 19.2% of paclitaxel was released from the P-CMs at 12 h, which was significantly lower than that released from P-NCMs (41.1%). It is expected that the crosslinking structure could not completely halt drug release from the micelles, however, these results support the notion that the polydisulfide crosslinks fortify the micellar structure and allow the crosslinked PEG_{5K}-VE₄-TA₄ micelles to better retain the incorporated drug molecules within the micellar core.

To study the release of the drug-loaded crosslinked micelles in response to reduction, the release study of P-CMs in PBS with the addition of 2 µM or 10 mM GSH, which corresponds to the extracellular and intracellular GSH levels, respectively, was studied. As shown in FIG. **5B**, the presence of 2 µM GSH in the micellar solution did not alter the release profile of paclitaxel from P-CMs, pointing to excellent stability of the polydisulfide crosslinks in the non-reducing environment found in the circulation and tissue fluids. When GSH concentration was increased to 10 mM, 59.4% of the drug was released from P-CMs at 12 h, which was even faster than that of P-NCMs in the absence of GSH. The rapid release of P-CMs could be attributed to prompt breakdown of the disulfide linkages by GSH reduction. The resulting free thiols, highly hydrophilic groups, could drastically decrease the hydrophobic interactions inside the micellar core, leading to accelerated drug release. This finding is also consistent with the above stability study (FIG. **4D**) showing that P-CMs quickly lost structural integrity in PBS buffer containing 10 mM GSH. These results indicate that the crosslinking of micelles is reversible in the reducing environment prevalent in the cytoplasm of tissue cells.

### Intracellular Uptake of the Crosslinked PEG_{5K}-VE₄-TA₄ Micelles

A fluorescence resonance energy transfer (FRET) pair of hydrophobic dyes, DiO (Ex/Em 488/501 nm) and DiI (Ex/Em 501/565 nm), was used to investigate the uptake of the crosslinked PEG_{5K}-VE₄-TA₄ micelles into the tumor cells. FRET occurs when a donor dye (DiO) and an acceptor dye (DiI) are present within the range of Förster distance, the emission fluorescence from the excited donor dye is used as the excitation energy for the acceptor dye, resulting in the emission of the acceptor fluorescence. Since both DiO and DiI are encapsulated in the micellar core within Förster distance, when the micelles are exited at 488 nm, the emission energy from DiO can be transferred to adjacent DiI, which subsequently gets excited and emits the fluorescence at 565 nm. In contrast, when the micelles disintegrate or the dye molecules are released from the micelles, no fluorescence from DiI is emitted due to the lack of excitation energy. This feature could therefore be exploited to monitor the intracellular uptake of the intact micelles along with the entrapped payload.

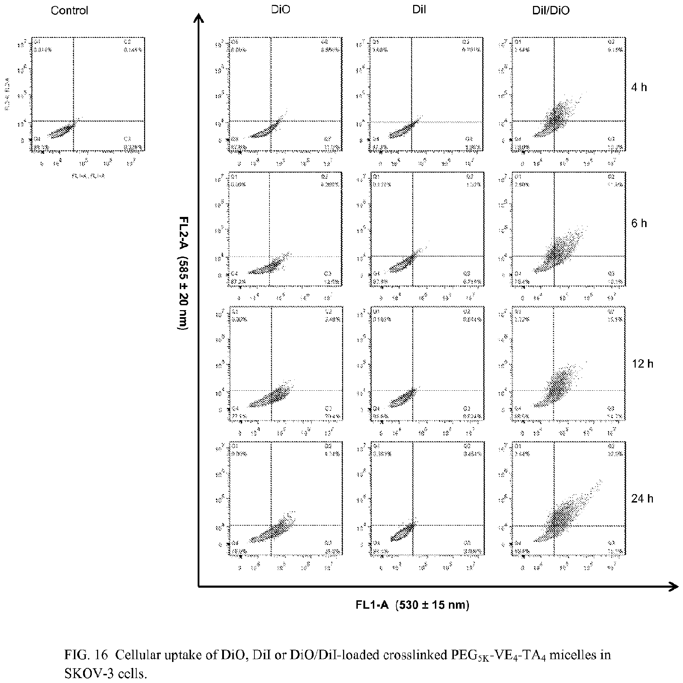

The intracellular uptake of the crosslinked PEG_{5K}-VE₄-TA₄ micelles was studied by directly determining the fluorescence signal of DiO/DiI-loaded micelles in human ovarian cancer SKOV-3 cells. As shown in FIG. **6A** and FIG. **16**, when excited at 488 nm, the cells incubated with either DiO- or DiO/DiI-loaded micelles displayed strong green fluorescence signal at 530 ± 15 nm; whereas only the cells incubated with DiO/Dil-dual loaded micelles emitted red fluorescent signal at 585 ± 20 nm, no fluorescence signal was detected in the cells treated with DiI-loaded micelles. There was clear increase in both green (FL1) and red fluorescence (FL2) over 24-h incubation period. Because the emission of red fluorescence by DiI requires excitation energy transferred from adjacent DiO, these results strongly suggested that the crosslinked PEG_{5K}-VE₄-TA₄ micelles are internalized into the tumor cells with both dyes enclosed inside the micellar core. Similar results could be observed from the histogram analysis of the fluorescence intensity (FIGS. **6B** and **6C**). Compared to the rising green fluorescence intensity emitted from DiO over time, the increase in the red fluorescence from DiI was less drastic. This could be explained by rapid de-crosslinking of the micelles in the cytoplasm, resulting in less accumulation of DiI signal owing to the dissociation of the dye molecules from the micelles. Combined, these results strongly imply that the crosslinked PEG_{5K}-VE₄-TA₄ micelles can remain structurally intact and are taken up by the tumor cells along with the payload.

### Cytotoxicity of the Blank and Paclitaxel-Loaded PEG_{5K}-VE₄-TA₄ Micelles

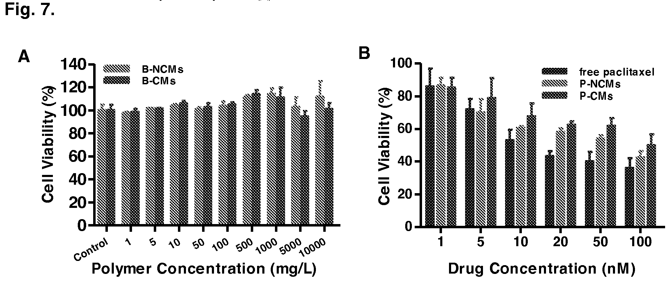

To serve as a safe drug carrier system, it is desirable that the empty micelles are inert and devoid of cytotoxicity. Since all of the building blocks of the PEG_{5K}-VE₄-TA₄ copolymer are nontoxic in nature, the copolymer itself is expected to cause minimal cytotoxicity. To this end, the cytotoxicity of the blank non-crosslinked (B-NCMs) and the blank crosslinked micelles (B-CMs) against SKOV-3 cells was examined. There was little cytotoxicity for either micelle type even at a polymer concentration up to 10 mg/mL (see FIG. **7A**), signifying the safety and biocompatibility of the PEG_{5K}-VE₄-TA₄ copolymer.

Subsequently, the cytotoxicity of paclitaxel-loaded non-crosslinked (P-NCMs) and crosslinked micelles (P-CMs) was examined in SKOV-3 cells at paclitaxel concentration ranging from 1-100 nM for 72 h (see, FIG. **7B**). Both micellar formulations displayed lower antiprolierative potency than free paclitaxel, which could be ascribed to the deterred drug release from the micelles. While the free drug could rapidly and passively diffuse into the tumor cells to exert the activity, the drug-loaded micelles could be only internalized into the cells via energy-dependent endocytosis, which is a slower and less efficient process than passive diffusion.

### Pharmacokinetics of Paclitaxel-Loaded PEG_{5K}-VE₄-TA₄ Micelles in Mice

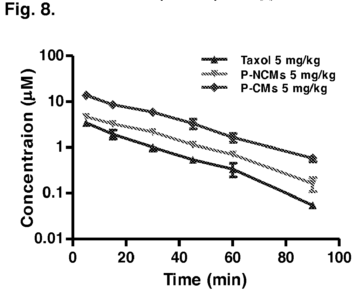

In the clinic, paclitaxel is solubilized in Cremophor EL-based formulation (Taxol®) prior to intravenous administration. To evaluate the therapeutic utility of the crosslinked PEG_{5K}-VE₄-TA₄ micelles as a nanocarrier system for the delivery of paclitaxel, the pharmacokinetics of P-CMs, P-NCM and Taxol® at an identical dose of 5 mg/kg paclitaxel was examined. As shown in FIG. **8** and Table 2 below, the plasma concentration of paclitaxel from all three formulations declined in parallel with indistinguishable elimination half-lives (t_{1/2,e}), indicating similar elimination kinetics of paclitaxel regardless of the dosage forms.

**Table 2. Pharmacokinetic Parameters of Paclitaxel Following Intravenous Administration of Taxol®, P-NCMs and P-CMs in Mice at an Identical Dose of 5 mg/kg Paclitaxel**

| Parameters | Taxol® | P-NCMs | P-CMs |
|---|---|---|---|
| t_{1/2,e} (min) | 14.2 ± 1.1 | 16.7 ± 0.7 | 17.2 ± 2.2 |
| AUC (µM·min) | 78.3 ± 9.5 | 139.6 ± 6.9 | 385.1 ± 33.5 |
| CL_{T} (ml/kg/min) | 74.4 ± 8.4 | 40.8 ± 2.4 | 14.8 ± 1.2 |
| Vd,ss (ml/kg) | 1521.6 ± 235.6 | 982.8 ± 12.8 | 366.8 ± 74.4 |

The results shown in Table 2 represent the mean ± SD (n=3).

Importantly, intravenous administration of P-CMs resulted in approximately 5-fold and 3-fold higher paclitaxel level in plasma than that of Taxol® and P-NCM, respectively. Non-compartmental analysis on these profiles revealed that compared to Taxol®, the incorporation of paclitaxel within the PEG_{5K}-VE₄-TA₄ micelles reduced the volume of distribution (V_{d,ss}) of paclitaxel by 35% for P-NCMs and 76% for P-CMs. Consequently, the total clearance (CL_{T}) of P-NCM and P-CMs was 45% and 80% of Taxol®, respectively. These results clearly indicate that the PEG_{5K}-VE₄-TA₄ micelles are able to retain paclitaxel within the micellar core and restrict the drug distribution into the extravascular space, whereas the crosslinking of the micelles further improves the drug retention within the micelles and prolong the circulation time of paclitaxel.

### In vivo Biodistribution and Tumor Accumulation of DiD/Paclitaxel-Dual Loaded PEG_{5K}-VE₄-TA₄ Micelles in Mice

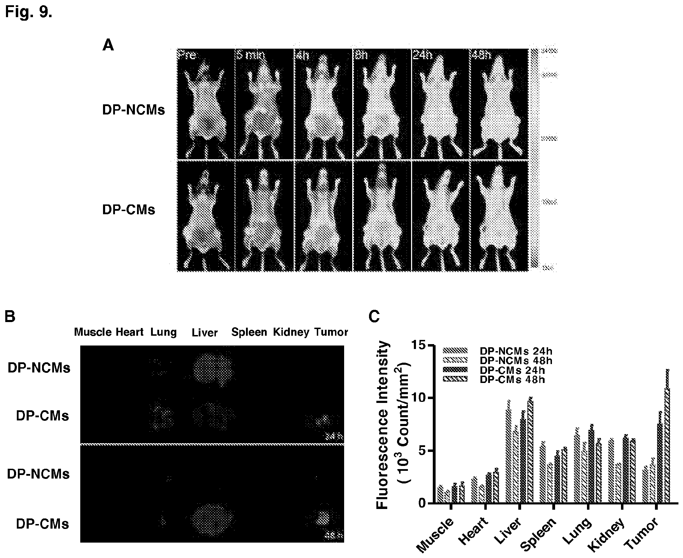

To further investigate the pharmacokinetic characteristics of paclitaxel-loaded PEG_{5K}-VE₄-TA₄ micelles, DiD, a hydrophobic near infrared (NIR) fluorescent dye, which enables whole-body imaging with deep tissue penetration and low tissue absorption/scattering, was co-loaded with paclitaxel in the non-crosslinked (DP-NCMs) and the crosslinked (DP-CMs) micelles. The real-time *in vivo* biodistribution of DP-NCMs and DP-CMs by employing NIR imaging over 48 h was monitored. As shown in FIG. **9A**, the NIR fluorescence signal was immediately detectable throughout the body following intravenous injection of both formulations because of rapid mixing of DP-NCMs and DP-CMs in the bloodstream. The contrast of the DiD signal in the tumors vs. the rest of the body became noticeable since 4 h post injection, suggesting preferential accumulation of the PEG_{5K}-VE₄-TA₄ micelles in the tumor. The DiD intensity in the tumors treated with DP-CMs was higher than that of DP-NCMs, a clear indication of better tumor extravasation for DP-CMs, which is consistent with improved kinetic stability of P-CMs in serum (see, FIG. **4C**) and the prolonged circulation time of P-CMs *in vivo* as described above (FIG. **8**). Notably, there was higher NIR fluorescent intensity in the chest area for the non-crosslinked micelles than that of the crosslinked micelles at 24-48 h, likely due to the more extensive distribution of free DiD released from DP-NCMs than that of DP-CMs, which is consistent with the pharmacokinetic analysis showing the much reduced V_{d,ss} of P-CMs compared to that of P-NCMs.

Next, the normal organs and tumor tissues were dissected for NIR imaging (see, FIG. 9B) and the fluorescence intensity was quantified (see, FIG. **9C**). The DiD signal varied among the organs with the lowest found in the muscle and heart tissues for both formulations. There was a general trend that the DiD intensity in the well-perfused organs (i.e., liver, spleen, lung, heart and kidney) decreased from 24 h to 48 h in the DP-NCMs-treated mice, whereas the NIR signal remained same or slightly increased post DP-CMs injection. This may be rationalized by the fact that the crosslinked micelles remain intact in the circulation for a longer period of time than the non-crosslinking counterparts, resulting in more extended exposure of the payload in the well-perfused organs. Treatment of DP-NCMs and DP-CMs yielded similar fluorescence intensity in the majority of normal organs at 24 h. There was higher NIR signal in the DP-CMs-treated organs at 48 h, but the difference between the two micelle types in each corresponding normal organ was rather modest (< 100% increase). By contrast, the NIR intensity of the DP-CMs-treated tumors was 2.4-fold and 3.0-fold higher than that of the DP-NCMs-treated tumors at 24 h and 48 h, respectively. These findings strongly suggest that the crosslinking of PEG_{5K}-VE₄-TA₄ micelles alters the biodistribution of the payload and preferentially enhances drug accumulation in the tumor via the EPR effect.

### Anticancer Efficacy of Paclitaxel-Loaded PEG_{5K}-VE₄-TA₄ Micelles in Tumor-Bearing Mice

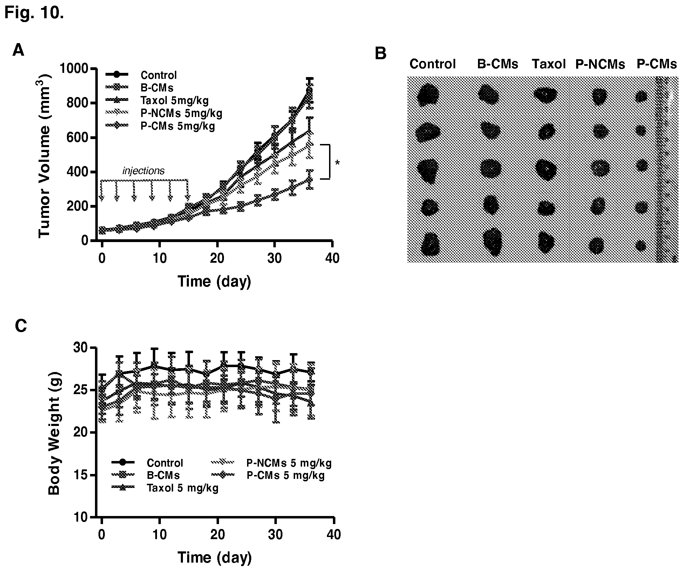

The major toxicities associated with Taxol® therapy include the hypersensitivity reactions induced by Cremophor EL and peripheral neuropathy caused by paclitaxel. It will be clinically beneficial to develop an alternative formulation for paclitaxel that: (1) requires no Cremophor EL as a solubilizer; and (2) is amenable to a more frequent and lower dosing regimen to maximize the therapeutic window of paclitaxel. Encouraged by the promising pharmacokinetic results demonstrating that the encapsulation of paclitaxel within the PEG_{5K}-VE₄-TA₄ micelles notably elevated the plasma concentration of paclitaxel and the crosslinking of the micelles further prolonged the circulation time of paclitaxel, the anticancer efficacy of PCMs and P-NCMs in the tumor-bearing mice was evaluated. Although Taxol® is found to be efficacious at a weekly dose of 20 mg/kg, the mice were treated with twice-weekly 5 mg/kg paclitaxel to observe possible potentiation of its anticancer efficacy via the micellar formulations. As shown in FIGS. **10A** and **10B**, SKOV-3 tumors in the untreated or B-CMs-treated groups grew rapidly over the 5-week study period, whereas Taxol® modestly slowed down the tumor growth compared to the untreated group (p =0.034 on day 35) and P-NCMs exerted tumor growth inhibition similar to Taxol®. In contrast, P-CMs suppressed tumor growth with higher potency than P-NCMs and Taxol®, which became statistically significant starting day 24 (*p* < 0.01). None of the treated mice experienced overt toxicity, as manifested by the steady body weight in all groups for 5 weeks (see, FIG. **10C**).

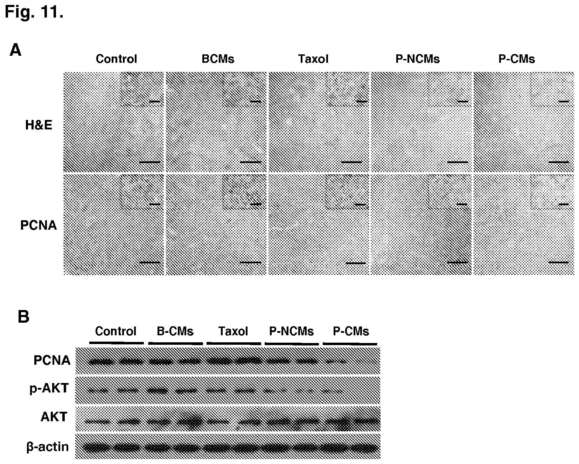

To further evaluate the *in vivo* anti-proliferative effect of P-CMs, the tumor specimens were examined by histological analysis and immunohistochemistry. H&E staining showed that P-CMs-treated tumor tissues had smaller tumor cells with less nuclear division when compared to the rest groups (see, FIG. **11A**). Immunohistochemistry staining with PCNA, a nuclear protein and a cell proliferation marker, revealed that the tumor cells in the P-CMs-treated mice underwent the least proliferation, whereas high proliferation activity was observed in the tumors of the untreated, Taxol® and B-CMs-treated mice (FIG. **11A**). This result was further confirmed by Western blot analysis (see, FIG. **11B**), showing the lowest PCNA protein levels in P-CMs-treated tumors. In addition, p-Akt level was markedly reduced in the P-CMs-treated tumors, correlating well with the lower proliferative propensity of the tumor cells than that of the rest groups. Collectively, the above findings provide strong evidence that the crosslinked PEG_{5K}-VE₄-TA₄ micelles greatly potentiate the anticancer efficacy of paclitaxel *in vivo.*

In summary, synthesized redox-responsive PEG_{5K}-VE₄-TA₄ copolymers were synthesized. The PEG_{5K}-VE₄-TA₄ copolymer readily formed micelles in aqueous solution and can be further crosslinked by a catalytic amount of DTT. The crosslinked PEG_{5K}-VE₄-TA₄ copolymer micelles demonstrated improved thermodynamic and kinetic stability, which significantly augmented the delivery of paclitaxel to the tumor tissue and enhanced its antitumor efficacy a human ovarian cancer murine model. Constructed from all naturally occurring, biodegradable and/or biocompatible components, this novel crosslinked micellar nanocarrier system represents an interesting and promising platform for the delivery of sparsely aqueous soluble anticancer agents.

### Example 2 - Preparation and Use of PEG:VE:DET Copolymer

In this example of the present invention, a novel cationic copolymer comprised of polyethylene glycol 5000 (PEG_{5K}), Vitamin E (VE) and diethylenetriamine (DET) at 1:4:20 molar ratio was synthesized. The resulting PEG_{5K}-VE₄-DET₂₀ copolymer assembled into hybrid micelles when mixed with neutral PEG_{5K}-VE₄ copolymer at a 1:8 weight ratio, which were investigated as the nanocarriers for combined delivery of paclitaxel and *let-7b* mimic. It was determined that the PEG_{5K}-VE₄-DET₂₀ hybrid micelles could retain paclitaxel for an extended period and burst release the drug in the presence of cathepsin B, demonstrating the biodegradability of the copolymers. At a N/P ratio of 12:1, the PEG_{5K}-VE₄-DET₂₀ hybrid micelles formed stable polyplexes with *let-7b* mimic, which were efficiently taken up by tumor cells and underwent endosomal escape. In non-small cell lung cancer A549 cells that harbor mutant *KRAS,* paclitaxel and *let-7b* mimic-dual loaded micelles (M-PTX/*let-7b*) markedly potentiated the cytotoxicity of paclitaxel, elevated apoptosis and diminished the invasiveness of tumor cells. In mice bearing subcutaneous A549 xenografts, intravenous administration of M-PTX/*let-7b* retarded tumor growth more potently than Taxol®. The present example demonstrated the promise of PEG_{5K}-VE₄-DET₂₀ hybrid micelles for concurrent delivery of hydrophobic drugs and miRNA mimics.

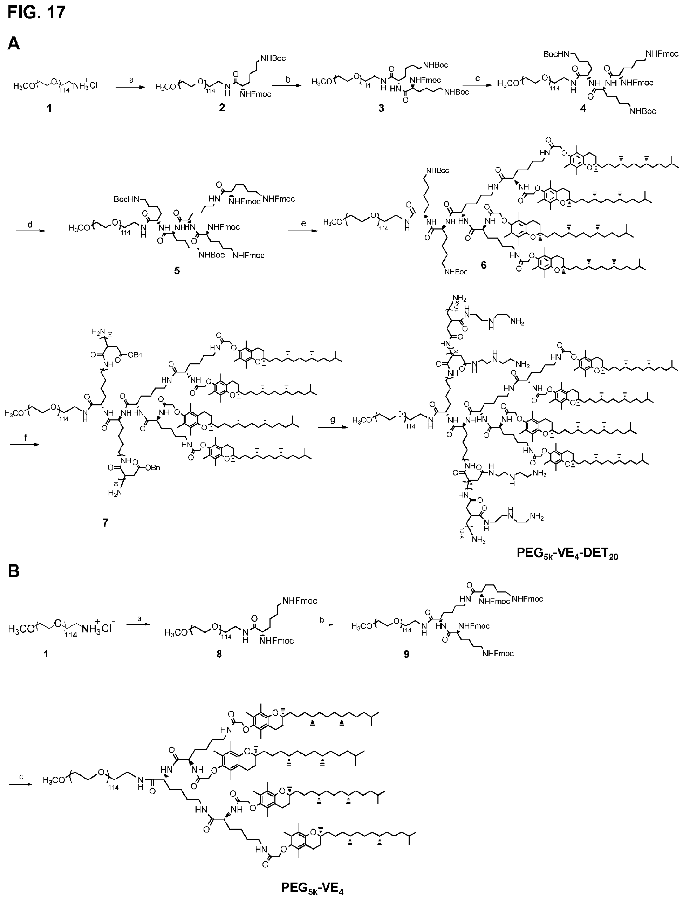

While PEG_{5K} provided the hydrophilic palisades that confer the stealth property by sterically hindering protein adsorption, the aliphatic chain and aromatic ring of VE aggregated into the hydrophobic core that stabilized the micellar structure and greatly increased the aqueous solubility of hydrophobic drugs. At a 1:4 molar ratio, the conjugation of PEG_{5K} with VE via polylysine linkage resulted in an amphiphilic copolymer that could self-assemble into micelles with excellent loading capacity for paclitaxel, an otherwise sparingly water-soluble drug that requires Cremophor EL and ethanol for solubilization. Two polyasparagine chains carrying N-(2-aminoethyl) aminoethyl groups (DET₁₀), which are known to be protonated at physiological pH and form polyplexes with nucleic acids, were grafted onto PEG_{5K}-VE₄ via peptide linkage. The resulting PEG_{5K}-VE₄-DET₂₀ copolymer readily formed hybrid micelles with the neutral PEG_{5K}-VE₄ copolymer that had superior solubilizing and loading capacity for paclitaxel via hydrophobic interactions, and formed stable polyplexes with *let-7b* mimic via electrostatic forces (See, the reaction scheme shown in FIG. **17A**). Paclitaxel and *let-7b* mimic-dual loaded micelles (M-PTX/*let-7b*) were evaluated for drug release, cellular internalization and chemosensitization in human non-small cell lung cancer A549 cells that carry KRAS^{G12S} mutation. The *in vivo* efficacy of M-PTX/*let-7b* was investigated in mice bearing A549 xenografts following intravenous administration.

### Synthesis of PEG_{5K}-VE₄-DET₂₀

PEG_{5K}-VE₄-DET₂₀ copolymer was synthesized via stepwise peptide condensation reactions from MeO-PEG-NH₂·HCl as depicted in FIG. **17A**. PEG_{5K}-NH₂ **1** (2 g, 0.4 mmol) was coupled by Fmoc-Lys(Boc)-OH (375 mg, 0.8 mmol) in the presence of DIC (150 µL, 0.96 mmol), HOBt (164 mg, 0.96 mmol) and DIEA (350 µL, 2 mmol) in DMF (50 mL) for 3 h at room temperature. The reaction mixture was poured into diethyl ether (200 mL) until the completion of the coupling reaction indicated by the Kaiser test. The precipitates were collected after centrifugation, washed by fresh ether (3×150 mL) and dried under vacuum to yield PEGylated compound **2**. Fmoc groups were deprotected by the treatment with piperidine in DMF (v/v 1:4, 100 mL). The PEGylated compound was precipitated and washed with cold ether (200 mL). One more coupling of Fmoc-Lys(Boc)-OH and two more coupling of Fmoc-Lys(Fmoc)-OH were carried out step by step to furnish PEGylated compound **5.** After the removal of Fmoc groups by piperidine in DMF, α-tocopheryloxyacetic acid synthesized according to a known method was conjugated to the amino terminals of the lysines to afford PEGylated compound **6**. The Boc groups of PEGylated compound **6** were removed by TFA in dichloromethane (v/v 1:3, 20 mL) for 30 min. The mixture was precipitated in cold ether and collected following centrifugation. The PEG_{5K}-VE₄-Asp₂₀ copolymer **7** was synthesized by further ring-opening polymerization of BLA-NCA initiated by the deprotection of compound **6**. The product was precipitated by diethyl ether and purified by repeated precipitation in diethyl ether and dried in vacuum. The PEG_{5K}VE₄-Asp₂₀ copolymer **7** was dissolved in DMF, followed by the reaction with diethylenetriamine DET (20 equivalents to the benzyl ester residue in PLBA) under mild anhydrous conditions at 40°C for 2 h to obtain PEG_{5K}-VE₄-DET₂₀, which was further purified by dialysis against deionized water for 2 days and was lyophilized to a pale yellow powder.

In summary, the reaction scheme shown in FIG. **17A** utilized to following reagents and reaction conditions in steps (a) to (f)(ii):
(a) Fmoc-Lys(Bos)-OH, DIC, HOBt, DIEA, DMF, r.t, 6 h.
(b)(i) 20% Piperidine in DMF, r.t, 2 h.
(b)(ii) Fmoc-Lys(Bos)-OH, DIC, HOBt, DIEA, DMF, r.t, 6 h.
(c)(i) and (d)(i) 20% Piperidine in DMF, r.t, 2 h.
(c)(ii) and (d)(ii) Fmoc-Lys(Fmoc)-OH, DIC, HOBt, DIEA, DMF, r.t, 6 h.
(e)(i) 20% Piperidine in DMF, r.t, 2 h.
(e)(ii) a-tocopheryloxyacetic acid, DIC, NHS, DIEA, DMF, r.t, overnight.
(f)(i) 50% TFA in CHCl₃, r.t, 1 h.
(f)(ii) BLA-NCA, DMF, r.t, 48 h. g, 20 equiv DET, DMF, 40°C, 2h.

### Synthesis of PEG_{5K}-VE₄

The PEG_{5K}-VE₄ copolymer was synthesized via stepwise peptide condensation reactions from MeO-PEG-NH₂·HCl as shown in the reaction scheme of FIG. **17B**. PEG_{5K}-NH₂ **1** (2 g, 0.4 mmol) was coupled by Fmoc-Lys(Fmoc)-OH (375 mg, 0.8 mmol) in the presence of DIC (150 µL, 0.96 mmol), HOBt (164 mg, 0.96 mmol) and DIEA (350 µL, 2 mmol) in DMF (50 mL) for 3 h at room temperature. The reaction mixture was poured into diethyl ether (200 mL) until the completion of the coupling reaction indicated by the Kaiser test. The precipitant was collected after centrifugation, washed by fresh ether (3×150 mL) and dried under vacuum to yield PEGylated compound **8**. Fmoc groups were deprotected by the treatment with piperidine in DMF (v/v 1:4, 100 mL). The PEGylated compound was precipitated and washed with cold ether (200 mL). One more coupling step of Fmoc-Lys(Fmoc)-OH were carried out to furnish PEGylated compound **9**. After the removal of Fmoc groups by piperidine in DMF, α-tocopheryloxyacetic acid was conjugated to the amino terminal of the lysine residues to afford the PEG_{5K}-VE₄ copolymer. The copolymer was further purified by dialysis against deionized water for 2 days and was lyophilized to a pale white powder.

In summary, the reaction scheme shown in FIG. **17B** utilized to following reagents and reaction conditions in steps (a) to (c)(ii):
(a) Fmoc-Lys(Fmoc)-OH, DIC, HOBt, DIEA, DMF, r.t, 6 h.
(b)(i) 20% Piperidine in DMF, r.t, 2 h.
(b)(ii) Fmoc-Lys(Fmoc)-OH, DIC, HOBt, DIEA, DMF, r.t, 6 h.
(c)(i) 20% Piperidine in DMF, r.t, 2 h.
(c)(ii) a-tocopheryloxyacetic acid, DIC, NHS, DIEA, DMF, r.t, overnight.

### 1H NMR Spectroscopy

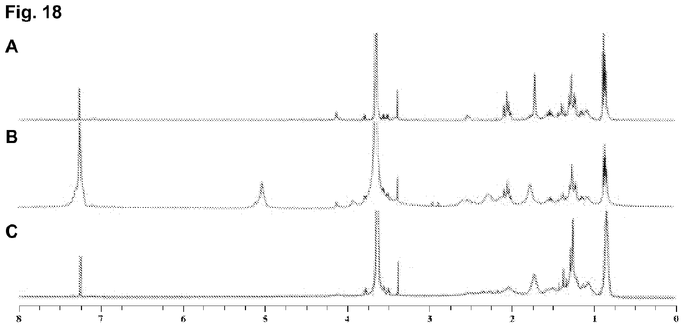

1H-NMR spectra of PEG_{5K}-VE₄-Asp₂₀, PEG_{5K}-VE₄-DET₂₀ and PEG_{5K}-VE₄ were collected on a 600 MHz Agilent Inova Spectrometer (Santa Clara, CA) at 20 °C using CDCl3 as a solvent as shown in FIGS. **18A-18C** respectively.

### Fabrication of the Blank and Drug-Loaded Micelles

### Preparation of the PEG_{5K}-VE₄-DET₂₀ Hybrid Micelles and Paclitaxel-Loaded Micelles (MPTX)

The PEG_{5K}-VE₄-DET₂₀ hybrid micelles were prepared by dry film method. Briefly, 90 mg copolymers consisting of 10 mg PEG_{5K}-VE4-DET₂₀ and 80 mg PEG_{5K}-VE₄ were dissolved in 2 mL chloroform in a 25-mL round-bottom flask, which was rotary evaporated to dryness at room temperature to form a homogenous thin drug-copolymer film. The resulting thin film was further dried overnight under vacuum to remove any residual solvent. The micelles were generated by hydrating the film with 1 mL phosphate buffered saline (PBS, pH 7.4) followed by 20 min sonication. Similarly, M-PTX were prepared by adding 0.43 mg of paclitaxel to the same amounts of copolymers prior to the dry film formation. The loading concentration of paclitaxel in the micelles, defined as the amount of the drug in the resulting micelle solution per unit volume of PBS, was quantified by high performance liquid chromatography (HPLC). The encapsulation efficiency of paclitaxel was calculated as the percentage of the incorporated versus the input paclitaxel amount.

### Preparation ofLet-7b Mimic-Loaded Micelles (M-let-7b) and M-PTX/let-7b

A predetermined amount (50 nmol) of *let-7b* mimic was added to the blank micelles or MPTX described above. The mixture was gently vortexed and then incubated at room temperature for 30 min. A series of *let-7b*-loaded micelles at different N/P ratios were prepared by varying the copolymer concentration. The N/P ratio is defined as the molar ratio between the total amine groups in PEG_{5K}-VE₄-DET₂₀ and the phosphate group in *let-7b* mimic. To evaluate the dissociation of *let-7b* mimic from the micelleplexes, the micelle solution was incubated at 37°C in the presence of heparin sulfate. The electrophoretic mobility of *let-7b* mimic was visualized on the Gel Doc™ XR⁺ system (Bio-Rad, Hercules, CA) with ethidium bromide staining after electrophoresis on a 1% (w/v) agarose gel for 10 min at 100 V in TAE buffer (40 mM Tris-HCl, 1% v/v acetic acid, 1 mM EDTA).

### Critical Micelle Concentration (CMC) of the PEG_{5K}-VE₄-DET₂₀ Hybrid Micelles

The CMC was determined by the fluorescence spectra using pyrene as a fluorescent probe. Pyrene solution (10 µL, 1 mM in ethanol) was added into the glass tubes, and the solvent was evaporated in darkness at room temperature. The micelle solution was diluted in deionized water to the total copolymer concentration ranging from 0.25-200 mg/L. Four mL of each was added to the pyrene-containing glass tubes and gently shaken for 24 h to reach equilibrium before measurement. The fluorescence spectra of pyrene were recorded by a fluorescence spectrophotometer (Perkin Elmer, Waltham, MA) at room temperature. The excitation wavelength at 331 nm and the emission fluorescence intensity at 373 nm and 384 nm were monitored. The CMC was estimated as the total copolymer concentration, where the intensity ratio I₃₇₃/I₃₈₃ begins to decrease markedly.

### Size and Surface Charge of the PEG_{5K}-VE₄-DET₂₀ Hybrid Micelles

The size distribution and zeta potential of the blank micelles, M-PTX, *M-let-7b* and *MPTX*/*let-7b* were assessed by dynamic light scattering (DLS) using a Nano Zetasizer ZS (Malvern Instruments, Malvern, UK). The micelle solution was diluted with deionized water to a final copolymer concentration of 0.5 mg/mL before the measurement. Each batch was analyzed in triplicates.

### Transmission Electron Microscopy (TEM)

The morphology of the micelles was observed at room temperature using a Tecnai G2 Spirit TEM (FEI, Hillsboro, OR). A drop of the micelle solution was deposited on a carbon-coated copper grid, dried at room temperature and negatively stained with 1% phosphotungstic acid before TEM visualization.

### The Stability of the PEG_{5K}-VE₄-DET₂₀ Hybrid Micelles

To evaluate the stability of *let-7b* mimic in serum, free *let-7b* mimic and M-PTX/*let-7b* (N/P ratio 12:1) were incubated in 10% (v/v) FBS at 37°C followed by the incubation with heparin sulfate for 45 min. The level of *let-7b* mimic was assessed by gel electrophoresis as described above. The stability of the PEG_{5K}-VE₄-DET₂₀ hybrid micelles in PBS was measured by monitoring size at 4°C for 4 weeks and the paclitaxel loading was assessed on HPLC.

### In vitro Release Study of Paclitaxel and Let-7b Mimic from M-PTX/let-7b

The release of paclitaxel and *let-7b* mimic from M-PTX/*let-7b* was studied in PBS (pH 7.4), or acetate buffered saline (pH 5.5) in the absence or presence of cathepsin B (10 units/mL) by using a dialysis method. The release of paclitaxel from Taxol® and free *let-7b* mimic were also studied as the controls. Briefly, the micelles were inserted into a Slide-A-Lyzer™ dialysis cassette (MWCO 20 kDa, Thermo Scientific, Rockford, IL), which was incubated in 50 mL of deionized water as the dialysis media at 37°C. At predetermined time points, the samples were withdrawn from the cassette, and the paclitaxel concentration was quantified by HPLC. The samples of the dialysis media were collected and lyophilized. The released *let-7b* mimic was quantified by quantitative real-time PCR (qRT-PCR). The dialysis media was refreshed at each time point to maintain the sink condition.

### HPLC Methodology

The concentration of paclitaxel was determined by a Waters HPLC system (Milford, MA) consisting of a 2795 pump with an autosampler, a 996 photodiode array detector and a Phenomenex (Torrance, CA) C18 column (5 µm, 4.6 mm × 150 mm). α-Naphthoflavone was used as an internal standard. Paclitaxel and α-naphthoflavone were detected at wavelengths of 227 nm and 281 nm, respectively. An isocratic mobile phase of 72% (v/v) methanol and 28% (v/v) sodium phosphate buffer (25 mM, pH 3.0) with 10 mM triethylamine was used at a flow rate of 1.2 mL/min.

### In vitro Cytotoxicity Assay

Human NSCLC cell line A549 (American Type Culture Collection, Manassas, VA) were grown in DMEM medium (Life Technologies) with 2 mM L-glutamine, which was supplemented with 10% FBS, 1% penicillin and streptomycin. The cells were maintained at 37°C with 5% CO₂ in a humidified incubator. To study the cytotoxicity of the blank PEG_{5K}-VE₄-DET₂₀ micelles, A549 cells were seeded at a density of 5,000-8,000 cells/well in 96-well plates and treated in triplicates with the micelles (total copolymer concentration 10-10⁴ mg/L), and the cell viability was determined after 72 h. Lipofectamine 2000 was used as a control. To evaluate the cytotoxicity of paclitaxel and *let-7b* mimic, A549 cells were treated in triplicates with varying concentrations of paclitaxel (1-25 nM) either in the free form, M-PTX or *M-PTX*/*let-7b* (*let-7b* mimic 50 nM) for 72 h. Cells were then fixed with 1% glutaraldehyde, stained with 0.1% crystal violet, and dissolved in 10% acetic acid. The absorbance was quantified at 595 nm on FLUOstar Omega plate reader (Cary, NC). The relative cell viability was calculated as the percentage of absorbance of the treated versus the untreated wells.

### Cellular Uptake and Transfection Efficiency

The uptake of M-PTX/*let-7b* into tumor cells was visualized by a laser scanning confocal fluorescence microscope coupled with using Live cell Imaging System (Zeiss, Jena, Germany). A549 cells (1×105) were seeded 24 h prior to the treatment. The endosomes/lysosomes in the cells were stained with LysoTracker Deep Red according to the manufacturer's instruction. Cy3-labeled *let-7b* mimic and Oregon green-labeled paclitaxel-loaded micelles (N/P ratio 12:1) were gently mixed and incubated at 37°C for 30 min to form micelleplexes, which were subsequently added to the cells. The cultured cells were monitored by for 48 h, and the fluorescence images of cells were captured by the laser scanning confocal fluorescence microscope using identical imaging parameters. The transfection efficiency of the PEG_{5K}-VE₄-DET₂₀ micelles was determined at different N/P ratios with Lipofectamine 2000 as the positive control. A549 cells (2×105 cells/well) were seeded in a 6-well plate and transfected with M*-let-7b* at a final concentration of 50 nM for 24 h. The transfected cells were then harvested, and the total RNA and proteins were extracted using the TRIzol Reagent (Life Technologies) and RIPA buffer, respectively. The *let-7b* mimic concentration was quantified by qRT-PCR, and KRAS protein was analyzed by Western blotting.

### Colony Formation Assay

A549 cells were treated with the blank micelles, M-PTX, M*-let-7b* and M-PTX/*let-7b* at a final concentration of *let-7b* mimic at 50 nM and paclitaxel at 20 nM for 48 h. The treated cells were harvested and re-seeded in triplicates in 12-well plates at a density of 150 cells/well. The cells were incubated for 14 days. The adherent cell colonies were fixed with 1% glutaraldehyde and stained with 0.1% crystal violet, imaged, and counted using an inverted microscope (Olympus, Tokyo, Japan). Experiments were independently repeated three times.

### Apoptosis Assay

Apoptosis was assessed by detecting the externalization of phosphatidyl serine using Annexin V Apoptosis Detection Kit (BD Biosciences, San Jose, CA). Following the treatments as described above, A549 cells were harvested and stained with Annexin V-FITC and propidium iodide (PI). After incubation for 15 min at room temperature, the samples were analyzed using an Accuri C6 Flow Cytometer System (BD Biosciences).

### Invasion Assay

Cell invasion was evaluated using a Boyden chamber system with a polycarbonate membrane (8-µm pore size; Corning, Corning, NY), which was coated with matrigel (BD Bioscience). Following the treatments as described above, A549 cells (105 cells suspended in serum-free media) were seeded into the upper chamber insert, and the lower chamber was filled with DMEM with 10% FBS. After 40 h, the cells remaining on the upper membrane were removed with cotton wool, whereas the cells that had invaded through the membrane were stained with 1% glutaraldehyde and 0.1% crystal violet, imaged, and counted using an inverted microscope. Experiments were independently repeated three times.

### Antitumor Efficacy Study

All animal procedures were performed in strict accordance with the Guide for the Care and Use of Laboratory Animals of the National Institutes of Health. The protocol was approved by the Institutional Animal Care and Use Committee at Mercer University (Approval Number: A1105007). A549 cells (1 × 107 in 0.1 mL matrigel/DMEM mixture) were subcutaneously implanted in the flank region of 5-6 weeks old female athymic nude mice (*nu*/*nu,* Charles River, Wilmington, MA). When the tumor volumes reached 50-100 mm3, the mice were randomized into six treatment groups (n=5) to receive the following treatments through the tail vein injection twice weekly for four weeks: (A) PBS; (B) the blank micelles; (C) Taxol®; (D) M-PTX; (E) M*-let-7b*; and (F) M-PTX/*let-7b.* For group B, the mice received identical amount of copolymers as groups D and F. For groups C and D, the mice were administered with paclitaxel at 5 mg/kg. For groups E and F, the mice were administrated with *let-7b* at 1 mg/kg. The tumor size was measured using a caliper twice-weekly, and the tumor volume was calculated as 1/2 × length × width2. The body weight of all mice was also measured twice weekly. On day 42, the mice were euthanized and the tumors were excised and divided for immediate formalin fixation and flash freezing (-80°C).

### Histology and Immunohistochemistry (IHC)

Formalin-fixed and paraffin-embedded tumor specimens of 3- to 4-µm thickness were sectioned and stained with hematoxylin and eosin (H&E) and IHC staining of the proliferating cell nuclear antigen (PCNA) antibody following the standard protocols.

### RNA Extraction, cDNA Synthesis and qRT-PCR

Total RNA in cells was extracted using TRIzol. The PrimeScript RT Reagent Kit (Clontech, Mountain View, CA) was used for reverse transcription of cDNA according to the manufacturer's procedures. The reactions were incubated first at 16°C for 30 min, at 42°C for 30 min, and were then inactivated at 85°C for 5 min. The intracellular level of *let-7b* was examined by qRT-PCR using TaqMan miRNA probes (Applied Biosystems, Foster City, CA) and Premix Ex Taq™ kit (Clontech) employing an ABI 7500 RT-PCR system (Applied Biosystems), and all of the reactions were run in triplicates. The *let-7b* level of was normalized to that of *U6* snRNA (Applied Biosystems) as an internal control, a ubiquitously expressed small nuclear RNA. The PCR reactions were incubated at 95°C for 5 minutes, followed by 40 cycles of 95°C for 15 seconds and 60°C for 1 minute. A comparative threshold cycle (ΔC_{T}) method was used to compare each treatment with the internal control, and the values are expressed as 2⁻_{ΔΔCT}.

### Western Blotting Analysis

Cells or tumor tissue samples (approximately 40-60 mg per sample) were homogenized and lysed using the RIPA buffer supplemented with the protease and phosphatase inhibitors. Cell or tumor lysates containing 30 µg of total proteins were resolved onto 12% SDS-PAGE gels, and transferred onto the nitrocellulose membranes. Blots were probed with the antibodies for phospho-ERK1/2 (p-ERK1/2), ERK1/2, cleaved caspase-3, caspase-3, E-cadherin, vimentin, HMGA2, PCNA, phosphor-AKT (p-AKT) and AKT, all of which were from Cell Signaling (Danvers, MA). Antibodies for KRAS and β-actin were from Santa Cruz Biotechnology, Inc. (Dallas, TX).

### Statistical Analysis

All data are presented as the mean ± standard deviation (SD). Data from the different groups were compared using the Student's *t*-test. A *p*-value of less than 0.05 was considered statistically significant, as indicated by asterisks in the figures.

### Results and Discussion

### Synthesis of PEG_{5K}-VE₄-DET₂₀ and PEG_{5K}-VE₄ Copolymers

PEG_{5K}-VE₄-DET₂₀ and PEG_{5K}-VE₄ were synthesized via stepwise peptide condensation reactions using PEG_{5K}-NH₂ as the starting material, as shown in FIGS. **17A-****17B**. The completion of each reaction was confirmed by the Kaiser test and thin layer chromatography. The structures of the intermediate **7**, PEG_{5K}-VE₄-DET₂₀ and PEG_{5K}-VE₄ were confirmed by 1H NMR (FIGS. **18A-18C**). The average number of VE groups per PEG chain could be determined by comparing the peak intensity of the aliphatic protons of the terminal methyl group in VE at 0.8-0.9 ppm and the PEG methylene protons (-CH₂CH₂O-) at 3.7 ppm. The ratio of these two peaks showed that the numbers of VE groups per PEG chain was 3.85 and 4.1 in PEG_{5K}-VE₄-DET₂₀ and PEG_{5K}-VE₄, respectively. The alkyl peaks (0.8-1.4 ppm) and the phenylmethyl peak (2.1 ppm) in the intermediate 7 were found to be identical to those in PEG_{5K}-VE₄, indicating that the VE moiety remained intact during the Boc group deprotection by TFA. Since the absence of aromatic proton in 1H NMR spectrum of the PEG_{5K}-VE₄-DET₂₀ copolymer demonstrated that all the benzyl ester residues in the polymer backbone were substituted by DET. The polymerization degree of each polyasparagine chain could be determined by comparing the peak intensity at 5.09 ppm (methylene in the benzyl group) and 4.2 ppm (a-methylene neighboring to the carbonyl group of tocopheryloxyacetic acid), which was found to be 4.72. The molar ratio of PEG_{5K}:VE:DET was thus 1:3.85:18.18, which was in good agreement with the molecular formula of the target copolymer. The yield of PEG_{5K}-VE₄-DET₂₀ and PEG_{5K}-VE₄ were 51.3% and 63.7%, respectively, relative to the starting material PEG_{5K}-NH₂.

### Formulation Development of the PEG_{5K}-VE₄-DET₂₀ Hybrid Micelles

The PEG_{5K}-VE₄-DET₂₀ copolymer formed micelles readily in aqueous solution with an average diameter of 88 nm and a zeta potential of 30 mV. See, for example, the data in Table 3 below.

**Table 3. Characterization of the PEG_{5K}-VE₄-DET₂₀ Hybrid Micelles.**

| Micelles | PEG_{5K}-VE₄-DET₂₀: PEG_{5K}-VE₄ (w/w) | Size (nm) | PDI | Zeta potential (mV) | CMC (mg/L) |
|---|---|---|---|---|---|
| PEG_{5K}-VE₄-DET₂₀ | 1:0 | 88.3±1.4 | 0.58±0.016 | 30.3±1.6 | 10.75 |
| PEG_{5K}-VE₄ | 0:1 | 76.9±0.2 | 0.27±0.013 | 1.4±0.2 | 2.51 |
| PEG_{5K}-VE₄-DET₂₀ : PEG_{5K}-VE₄ | 1:1 | 80.7±1.2 | 0.53±0.019 | 28.1±0.6 | |
| | 1:2 | 75.4±2.1 | 0.43±0.014 | 24.2±0.9 | |
| | 1:4 | 72.9±0.4 | 0.34±0.056 | 19.8±0.4 | |
| | 1:8 | 60.8±0.1 | 0.20±0.014 | 12.0±0.7 | 2.77 |

Data represent the mean ± SD (n=3).

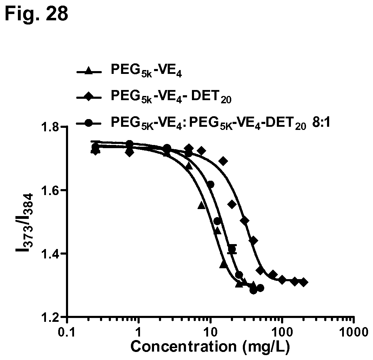

The CMC of the PEG_{5K}-VE₄-DET₂₀ micelles was 10.75 mg/L as shown in Table 3 and FIG. **28**. However, when tested for safety *in vivo,* the PEG_{5K}-VE₄-DET₂₀ micelles were found to cause fatality in mice following intravenous injection (data not shown). It is believed that the lethality was due to the highly positive surface charge of the micelles that is known to instantly bind to negatively charged cell membrane and proteins leading to the formation of aggregates in circulation, which may cause lung embolisms, complement activation and other acute toxicities. To decrease the surface charge, the cationic PEG_{5K}-VE₄-DET₂₀ copolymer was diluted with neutral PEG_{5K}-VE₄ copolymer at various ratios. As shown in Table 3, the zeta potential of the hybrid micelles decreased gradually as the proportion of PEG_{5K}-VE₄ was increased. When PEG_{5K}-VE₄-DET₂₀ and PEG_{5K}-VE₄ were mixed at 1:8 weight ratio, the resulting hybrid micelles had a surface charge of 12 mV and an average diameter of 60 nm. Moreover, the inclusion of PEG_{5K}-VE₄ notably decreased the polydispersity index (PDI) and the CMC of the hybrid micelles, indicative of enhanced thermodynamic stability of the nanoassemblies. This could be accounted by less repulsion from the cationic DET moieties as the hybrid micelles were formed. With the identical hydrophilic and hydrophobic blocks, PEG_{5K}-VE₄-DET₂₀ and PEG_{5K}-VE₄ were perfectly compatible in terms of micelle formation. The rest of our study used hybrid micelles using PEG_{5K}-VE₄-DET₂₀ and PEG_{5K}-VE₄ at a 1:8 weight ratio.

### Characterization of the PEG_{5K}-VE₄-DET₂₀ Micelleplexes

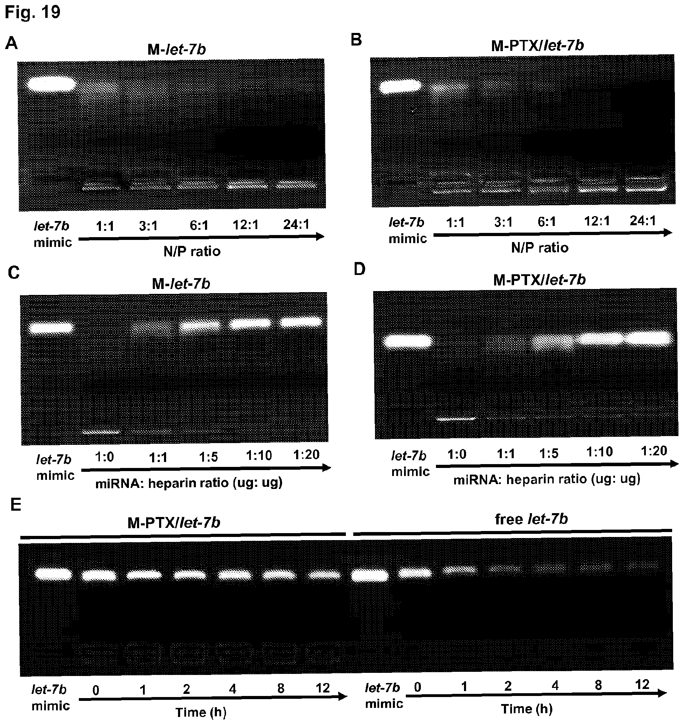

The formation of polyplexes between *let-7b* mimic and the PEG_{5K}-VE₄-DET₂₀ hybrid micelles was studied by a gel retardation assay. As shown in FIG. **19A**, naked *let-7b* mimic was small enough to migrate through the pores of 1% agarose gel upon electrophoresis, while the polyplexation of *let-7b* mimic with the micelles reduced *let-7b* mimic migration, with a complete retardation observed at N/P ratios above 12:1. The incorporation of paclitaxel in the micelle core did not interfere with the micelleplex formation (FIG. **19B**). To demonstrate the reversible nature of micelleplexes, excessive amounts of heparin, an endogenous polyanionic molecule, were incubated with the micelleplexes pre-formed at N/P ratio of 12:1. As shown in FIGS. **19C** and **19D**, a *let-7b* mimic:heparin weight ratio of 1:20 was sufficient to completely dissociate miRNA mimic from the M*-let-7b* or M-PTX/*let-7b* micelles. The incorporation of *let-7b* mimic into the micelles protected the miRNA mimic against serum degradation for a much longer duration compared to naked *let-7b* mimic, which was quickly cleaved by serum endonucleases (FIG. **19E**).

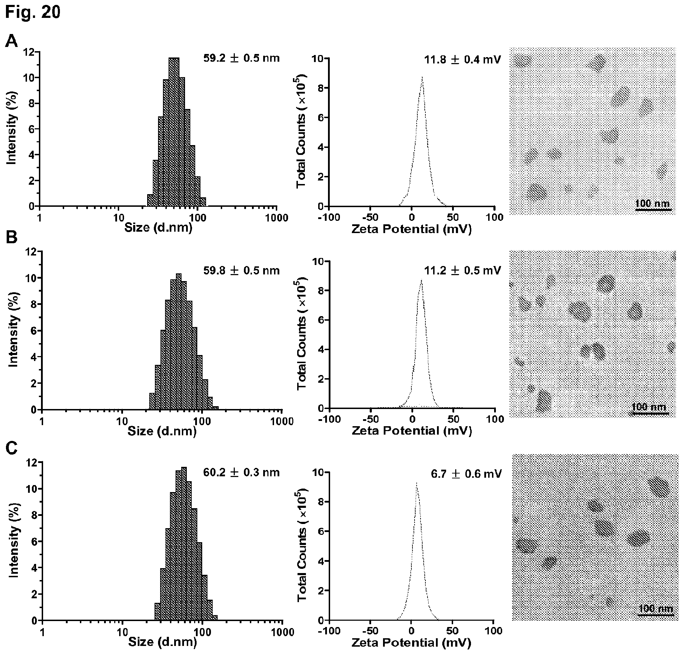

The size distribution and zeta potential of the blank hybrid micelles, M-PTX and M-PTX/*let-7b* micelles were shown in FIGS. **20A-20C** and summarized in Table 4 below.

**Table 4. Characterization of the Drug-Loaded PEG_{5K}-VE₄-DET₂₀ Hybrid Micelles**

| Micelles | PTX concentration (mM) | N/P | Size (nm) | PDI | Zeta potential (mV) |
|---|---|---|---|---|---|
| Blank micelles | | | 59.1±0.5 | 0.207±0.01 2 | 11.8±0.4 |
| M-PTX | 0.5 | | 59.8±0.5 | 0.195±0.02 1 | 11.2±0.5 |
| M*-let-7b* | | 12:1 | 56.4±0.3 | 0.182±0.00 2 | 6.9±0.2 |
| M-PTX/*let-7b* | 0.5 | 12:1 | 60.2±0.3 | 0.201±0.00 7 | 6.7±0.6 |

| | | | | | |
|---|---|---|---|---|---|
| M-PTX: paclitaxel-loaded PEG_{5K}-VE₄-DET₂₀ hybrid micelles *M-let-7b: let-7b* mimic-loaded PEG_{5K}-VE₄-DET₂₀ hybrid micelles M-PTX/*let-7b*: paclitaxel and *let-7b* mimic-dual loaded PEG_{5K}-VE₄-DET₂₀ hybrid micelles | | | | | |

Data represent the mean ± SD (n=3).

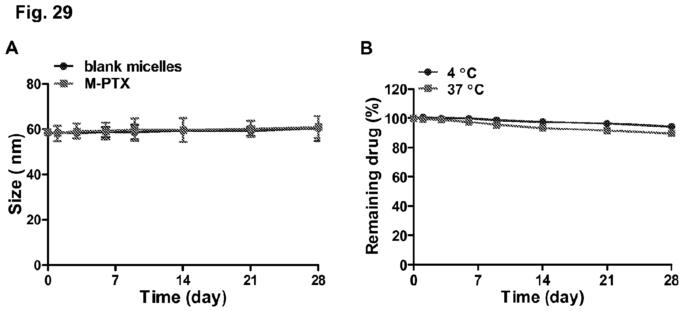

The incorporation of paclitaxel had negligible effect on the size (60 nm) and surface charge (11.2 mV) of the micelles. The polyplexation of *let-7b* mimic reduced the zeta potential of the micelles to about 7 mV, but minimally influenced the size. The hybrid micelles displayed excellent stability in PBS at 4°C for at least 4 weeks, reflected by little change in the size and the loading concentration of paclitaxel (FIG. **29**). The spherical morphology and the size distribution of the PEG_{5K}-VE₄-DET₂₀ hybrid micelles were confirmed by TEM (FIGS. **20A-20C**).

### Release of Paclitaxel and Let-7b Mimic from the PEG_{5K}-VE₄-DET₂₀ Micelleplexes

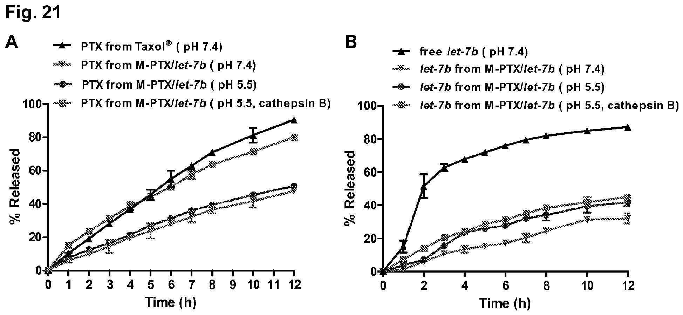

To assess the ability of the PEG_{5K}-VE₄-DET₂₀ hybrid micelles to function as a nanocarrier system for paclitaxel and *let-7b* mimic, the release kinetics of the payloads from M-PTX/*let-7b* was evaluated. As shown in FIG. **21A**, the release of paclitaxel from the M-PTX/*let-7b* (pH 7.4 or 5.5) was significantly slower than from the Cremophor EL/ethanol formulation (Taxol®), indicating better retention of paclitaxel within the PEG_{5K}-VE₄-DET₂₀ hybrid micelles. Likewise, the release of *let-7b* mimic from M-PTX/*let-7b* was markedly hampered compared to that of the naked *let-7b* mimic solution, indicating that the polyplexes formed with the DET₂₀ moieties reduce the diffusion of *let-7b* mimic (FIG. **21B**). The release of the payloads was further evaluated at pH 5.5 in the presence of cathepsin B, which simulates the acidic and enzymatic environment in the endosomes/lysosomes. Cathepsin B is a lysosomal protease overexpressed in the tumor tissues. It was found that the release of paclitaxel from the PEG_{5K}-VE₄-DET₂₀ hybrid micelles was greatly accelerated in the presence of cathepsin B, indicating the facile degradation of polylysine linker between PEG and VE blocks, resulting in the breakdown of the core-shell structure of the micelles and the burst release of paclitaxel. By contrast, the release of *let-7b* mimic from the micelles was little affected by cathepsin B, suggesting that DET₂₀-carrying polyasparagine is resistant to cathespin B.

### Cellular Uptake of the PEG_{5K}-VE₄-DET₂₀ Micelleplexes

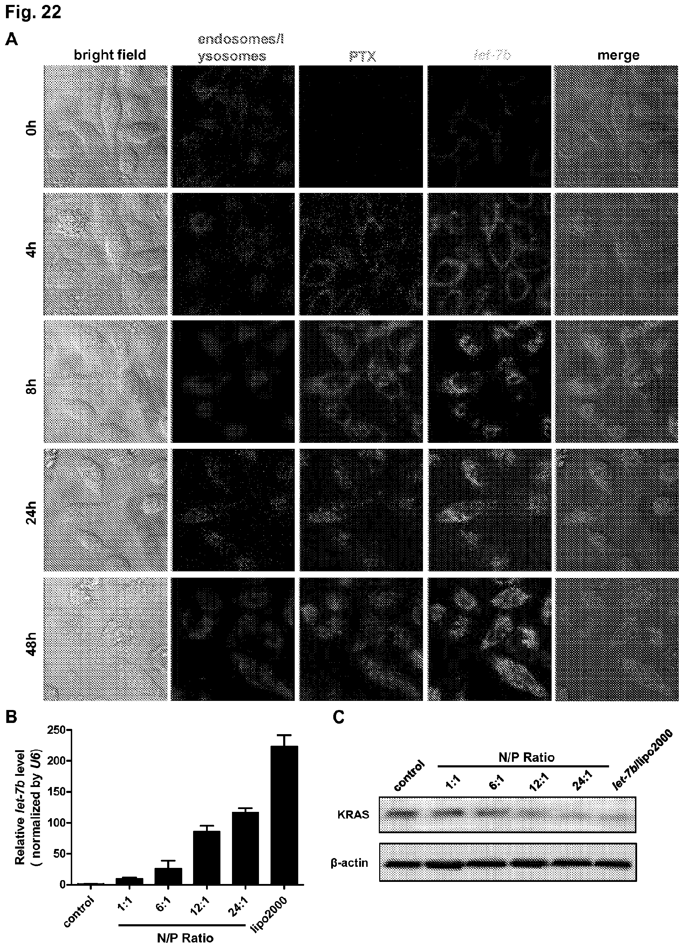

Endosomal escape is essential to achieve efficient transfection of nucleic acids. To track the uptake and intracellular fate of M-PTX/*let-7b*, confocal laser scanning microscope was employed to visualize Cy3-labeled *let-7b* mimic, Oregon green-labeled paclitaxel and LysoTracker Deep Red-labeled endosomes/lysosomes. As shown in FIG. **22A**, the cellular uptake of micelles occurred in a time-dependent manner. At 4 h, the micelles were mostly adhering to the cell surface with little cellular internalization. Extensive cellular uptake of the micelles was observed by 8 h. The intracellular signals of *let-7b* mimic and paclitaxel co-localized with that of the endosomes/lysosomes, consistent with the notion that the drug-loaded nanocarriers are taken up by the cells via endocytosis-mediated process. Interestingly, the fluorescence intensity of both *let-7b* mimic and paclitaxel became more diffused in the cytoplasm by 48 h, indicating the endosomal escape of these agents. When conjugated to polyasparagine, the ethylenediamine group (-NH-CH₂-CH₂-NH₂) of DET has pKa₁ at 9.5 and pKa₂ at 6.0, which undergoes two-step protonation in the acidic environment found in endosomes/lysosomes (pH 5). Similar to polyethylenimine, DET is shown to have a high capacity buffering endosomal acidification and cause an increase in osmotic pressure in the endosomes/lysosomes, leading to the disruption of the endosomal membrane to facilitate the release of nucleic acid into the cytoplasm, the so-called proton sponge effect.

*Let-7b* reconstitution in A549 cells treated with M*-let-7b* was further evaluated by qRT-PCR and Western blotting. As shown in FIG. **22B**, transfection of M*-let-7b* elevated the intracellular *let-7b* level in N/P ratio-dependent manner, resulting in over 100-fold increase at N/P ratio of 24:1. The transfection of *let-7b* mimic using Lipofectamine 2000 as a positive control raised the *let-7b* level by over 200-fold. To evaluate the biological function of *let-7b* repletion, the downregulation of *KRAS,* a direct target of *let-7* family, was assessed in A549 cells treated with M*-let-7b.* As shown in FIG. **22C**, at N/P ratios above 12:1, M*-let-7b* achieved *KRAS* protein suppression comparable to Lipofectamine 2000-mediated transfection. Together, these results indicated that the PEG_{5K}-VE₄-DET₂₀ micelleplexes efficiently deliver *let-7b* mimic to the cytoplasmic mRNA targets. This is in agreement with the literature that polyplex micelles bearing DET-linked polyasparagine as polycations are capable of delivering plasmid DNA and siRNAs with high transfection efficiency.

### Antiproliferative Activity of M-PTX/let-7b

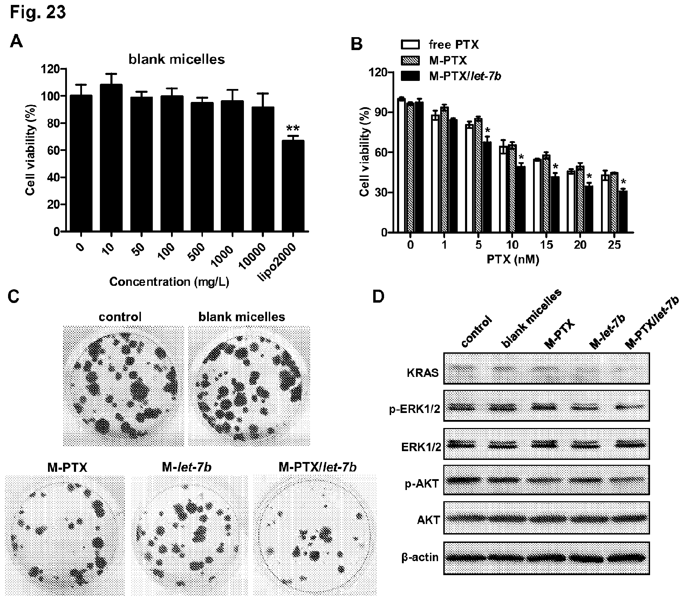

An important consideration when devising drug carrier systems is the minimal toxicity elicited by the carrier materials. To this end, the cytotoxicity of the blank PEG_{5K}-VE₄-DET₂₀ hybrid micelles in A549 cells was examined. It was found that the blank micelles were devoid of cytotoxicity at a total polymer concentration up to 10 g/L (FIG. **23A**). Meanwhile, the treatment of Lipofectamine 2000 for 3 days was notably more cytotoxic. Subsequently, the cytotoxicity of MPTX/*let-7b* was assessed with the concentration of *let-7b* mimic kept constant at 50 nM and the paclitaxel concentration ranging from 1-20 nM. While M-PTX displayed nearly identical cytotoxicity as free paclitaxel, the co-delivery of *let-7b* mimic with paclitaxel in M-PTX/*let-7b* clearly potentiated the cytotoxicity of paclitaxel (FIG. **23B**). Next, the colony formation assay was performed to evaluate the effect of M-PTX/*let-7b* on the long-term proliferative capacity of A549 cells. As shown in FIG. **23C**, the number and size of colonies formed by tumor cells was significantly shrinked following the treatment of the dual drug-loaded micelleplexes when compared to either agent alone.

As a driver oncogene, mutant KRAS constitutively activates MEK/ERK and PI3K/AKT signaling pathways, both of which are crucial to the survival and proliferation of tumor cells. To further investigate the molecular mechanism responsible for the antiproliferative effect of MPTX/*let-7b* in A549 cells that harbor *KRASG12S* mutation, the activation of ERK and AKT, two KRAS downstream effectors, was examined. As shown in FIG. **23D**, although the treatment of M*let-7b* and M-PTX/*let-7b* both ablated mutant KRAS protein similarly, M-PTX/*let-7b* reduced the phosphorylation of ERK1/2 and AKT to a much greater extent than *M-let-7b* or M-PTX. Together, these results indicate co-delivery of *let-7b* mimic and paclitaxel in the PEG_{5K}-VE₄-DET₂₀ hybrid micelles sensitizes *KRAS* mutant tumor cells to the antiproleferative activity of paclitaxel.

### Induction of Apoptosis by M-PTX/let-7b

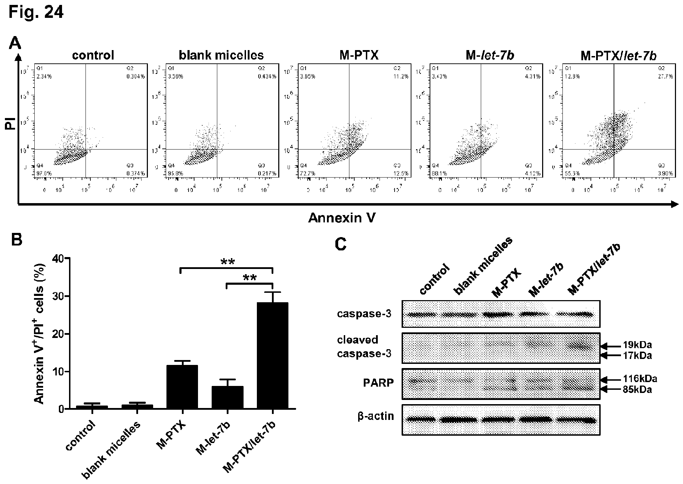

Apoptosis in A549 cells was evaluated by staining with Annexin V-FITC and propidium iodide (PI). Following 48 h incubation, M-*let-7b* and M-PTX induced late apoptotic cell population (Annexin V+/ PI+) by 4% and 11%, respectively. Strikingly, M-PTX-*let-7b* drastically increased late apoptotic cells to 27% (FIGS. **24A-24B**). The number of total apoptotic cells (Annexin V+) reflected the similar trend. Two apoptotic markers, the cleaved caspase-3 (19/17 KDa) and the cleaved PARP (85 KDa), were induced to higher levels by M-PTX-*let-7b* than either individual drug-loaded micelles (FIG. **24C**). These results indicate that co-delivery of *let-7b* mimic with paclitaxel via the PEG_{5K}-VE₄-DET₂₀ micelleplexes strongly induces apoptosis in *KRAS* mutant tumor cells.

### Blockage of Tumor Cell Invasion by M-PTX/let-7b

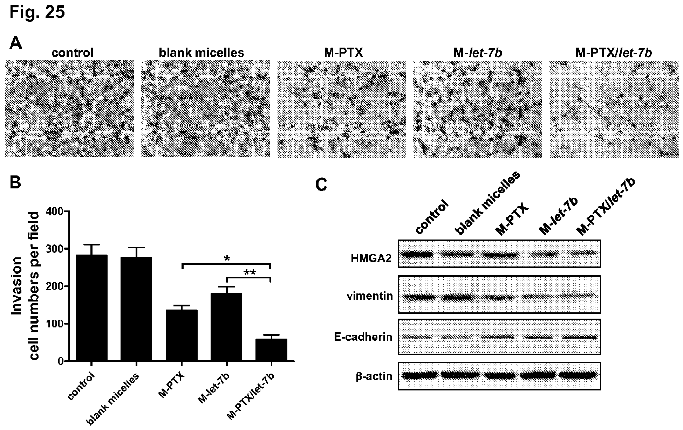

Epithelial-mesenchymal transition (EMT) converts adherent epithelial cells to motile mesenchymal cells, which is an important process during tumor invasion and metastasis. To evaluate the impact of M-PTX/*let-7b* on the invasiveness of tumor cells, the transwell invasion assay was carried out in A549 cells. It was found that the number of invading cells was diminished following the treatment of M-PTX/*let-7b*, whereas M-*let-7b* or M-PTX only modestly reduced invasion (FIGS. **25A-25B**). The expression of HMGA2, a direct target of *let-7* and a transcription factor involved in the regulation of cell motility, was downregulated by both M-*let-7b* and MPTX/*let-7b* (FIG. **25C**). Accordingly, the protein level of vimentin, a mesenchymal marker, was repressed in these cells. On the other hand, E-cadherin, an epithelial marker associated with cell adherence, were upregulated to a higher level in the cells treated with M-PTX/*let-7b* than either agent alone (FIG. **25C**). These results indicated that co-delivery of *let-7b* mimic with paclitaxel via the PEG_{5K}-VE₄-DET₂₀ micelleplexes blocks the invasion of *KRAS* mutant tumor cells and partially revert the EMT phenotype.

### Superior Antitumor Efficacy of M-PTX/let-7b in NSCLC Tumor-Bearing Mice

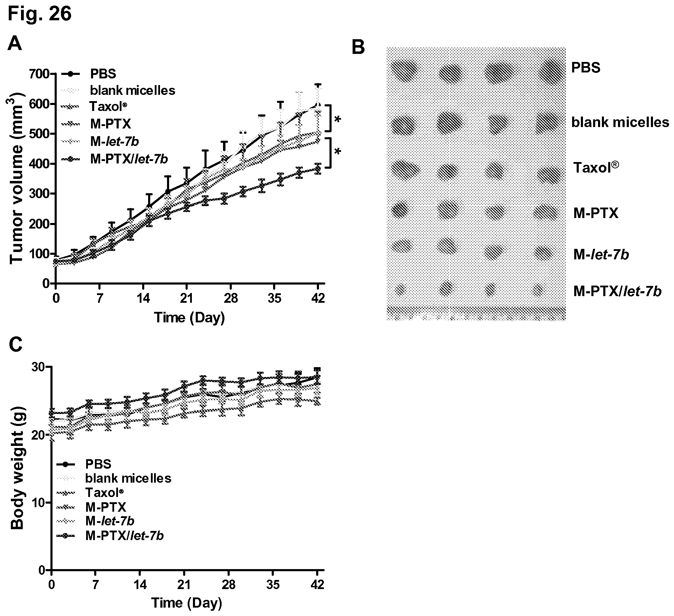

To explore the therapeutic application of the PEG_{5K}-VE₄-DET₂₀ hybrid micelles as nanocarriers for concurrent delivery of miRNA and chemotherapy *in vivo*, the antitumor efficacy of M-PTX/*let-7b* was studied in athymic mice bearing subcutaneous A549 xenografts. As shown in FIGS. **26A-26B**, A549 tumors grew rapidly over the 6-week period, which was minimally affected by the blank micelles. At twice-weekly 5 mg/kg, the treatment with Taxol® and M-PTX both modestly reduced the tumor burden, so did the treatment of M-*let-7b* at 1 mg/kg. Importantly, M-PTX/*let-7b* exerted the most potent tumor growth retardation compared to MPTX or M-*let-7b*, signifying superior anticancer efficacy associated with the combination therapy (*p* < 0.05). The body weight of the mice remained steady during the entire course of the study (FIG. **26C**), indicating no overt toxicity associated with any formulations.

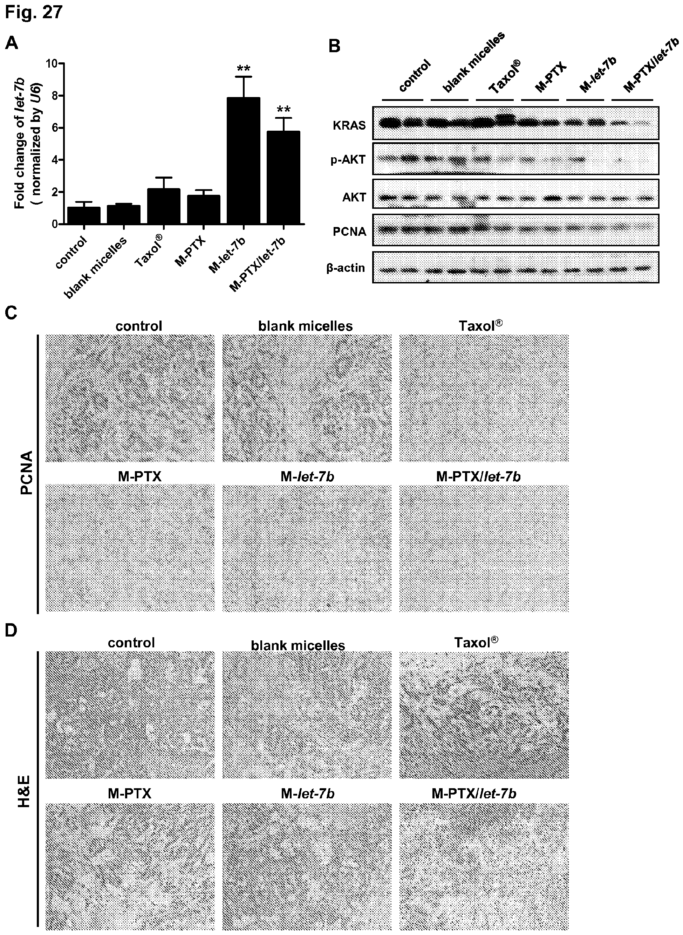

To further evaluate the therapeutic efficacy of paclitaxel/*let-7b* combination, the *let-7b* level in the tumor tissues by qRT-PCR was measured. It was found that, while neither Taxol® nor MPTX altered *let-7b* expression in the tumor, the treatment with M-*let-7b* or M-PTX/*let-7b* augmented the *let-7b* level by 5-7 fold (*p* < 0.01, FIG. **27A**). It is compelling that the elevated *let-7b* level had sustained for at least two weeks since the mice received the last dose of *let-7b* loaded micelles. The naked oligonucleotides are known to be rapidly eliminated from the circulation due to instant cleavage by serum nucleases as well as rapid renal excretion via glomerular filtration. The present data provided clear evidence that the PEG_{5K}-VE₄-DET₂₀ hybrid micelles stabilized *let-7b* mimic in the circulation and enabled the delivery of *let-7b* mimic to the tumor tissues. The tumor lysates were further analyzed by Western blotting, which showed lowest levels of KRAS and phospho-AKT in mice receiving the combination therapy (FIG. **27B**). The expression of PCNA, a cell proliferation marker, was repressed the most in M-PTX/*let-7b* treated tumors, revealed by IHC staining (FIG. **27C**) and confirmed by the Western blotting (FIG. **27B**). In addition, H&E staining of the tumor sections showed fewer and smaller tumor cells with larger necrotic areas in M-PTX/*let-7b-*treated tumors, when compared with the single drug-treated or untreated tumors (FIG. **27D**).

Associated with poor prognosis and drug resistance, *KRAS* mutations are highly prevalent in human cancers including NSCLC, pancreatic and colorectal cancers. Although molecularly targeted therapies are being developed to directly block mutant *KRAS*-driven oncogenesis, conventional chemotherapeutic drugs including paclitaxel are still the mainstay for treating *KRAS* mutant cancers. The present work provides proof-of-concept that *let-7* replacement therapy may be exploited to enhance the anticancer efficacy of paclitaxel in *KRAS* mutant cancers.

In summary, the herein-described self-assembling PEG_{5K}-VE₄-DET₂₀ hybrid micelles exhibited excellent transfection efficiency and minimal toxicity with particle size less than 100 nm. Paclitaxel and *let-7b* mimic dual loaded micelleplexes markedly potentiated the antiproliferative activity of paclitaxel, which was accompanied by increased apoptosis and reverted EMT phenotype in *KRAS* mutant tumor cells. In mice bearing *KRAS* mutant tumor xenografts, intravenous administration of M-PTX/*let-7b* resulted in significantly enhanced antitumor efficacy, demonstrating the PEG_{5K}-VE₄-DET₂₀ hybrid micelles as a robust platform for the dual delivery of hydrophobic drugs and miRNA mimics, as well as the therapeutic potential of *let-7b* mimic as a chemosensitizer for *KRAS* mutant cancers.

It should be understood that although the above-described amphiphilic copolymers, nanocarriers, compositions and/or methods are described as "comprising" one or more components or steps, the above-described amphiphilic copolymers, nanocarriers, compositions and/or methods may "comprise," "consists of," or "consist essentially of" the above-described components, features or steps of the amphiphilic copolymers, nanocarriers, compositions and/or methods. Consequently, where the present invention, or a portion thereof, has been described with an open-ended term such as "comprising," it should be readily understood that (unless otherwise stated) the description of the present invention, or the portion thereof, should also be interpreted to describe the present invention, or a portion thereof, using the terms "consisting essentially of" or "consisting of" or variations thereof as discussed below.

As used herein, the terms "comprises," "comprising," "includes," "including," "has," "having," "contains", "containing," "characterized by" or any other variation thereof, are intended to encompass a non-exclusive inclusion, subject to any limitation explicitly indicated otherwise, of the recited components. For example, an amphiphilic copolymer, a nanocarrier, composition and/or method that "comprises" a list of elements (e.g., components, features, or steps) is not necessarily limited to only those elements (or components or steps), but may include other elements (or components or steps) not expressly listed or inherent to the method and/or computing system.

As used herein, the transitional phrases "consists of" and "consisting of" exclude any element, step, or component not specified. For example, "consists of" or "consisting of" used in a claim would limit the claim to the components, materials or steps specifically recited in the claim except for impurities ordinarily associated therewith (i.e., impurities within a given component). When the phrase "consists of" or "consisting of" appears in a clause of the body of a claim, rather than immediately following the preamble, the phrase "consists of" or "consisting of" limits only the elements (or components or steps) set forth in that clause; other elements (or components) are not excluded from the claim as a whole.

As used herein, the transitional phrases "consists essentially of" and "consisting essentially of" are used to define an amphiphilic copolymer, a nanocarrier, composition and/or method that includes materials, steps, features, components, or elements, in addition to those literally disclosed, provided that these additional materials, steps, features, components, or elements do not materially affect the basic and novel characteristic(s) of the claimed invention. The term "consisting essentially of" occupies a middle ground between "comprising" and "consisting of".

Further, it should be understood that the herein-described amphiphilic copolymers, nanocarriers, compositions and/or methods may comprise, consist essentially of, or consist of any of the herein-described components, features and steps, as shown in the figures with or without any feature(s) not shown in the figures. In other words, in some embodiments, the amphiphilic copolymers, nanocarriers, compositions and/or methods of the present invention do not have any additional features other than those shown in the figures, and such additional features, not shown in the figures, are specifically excluded from the amphiphilic copolymers, nanocarriers, compositions and/or methods. In other embodiments, the amphiphilic copolymers, nanocarriers, compositions and/or methods of the present invention do have one or more additional features that are not shown in the figures.

While the specification has been described in detail with respect to specific embodiments thereof, it will be appreciated that those skilled in the art, upon attaining an understanding of the foregoing, may readily conceive of alterations to, variations of, and equivalents to these embodiments. Accordingly, the scope of the present invention should be assessed as that of the appended claims and any equivalents thereto.

## Claims

1. An amphiphilic copolymer comprising:
polyethylene glycol, and
three or more vitamin E units bonded to the polyethylene glycol.

2. The amphiphilic copolymer of claim 1, wherein said amphiphilic copolymer comprises from four to eight vitamin E units bonded to the polyethylene glycol.

3. The amphiphilic copolymer of claim 1 or 2, said amphiphilic copolymer having a structure: 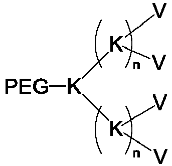 wherein PEG represents said polyethylene glycol, V represents a given vitamin E unit, K represents a first divalent linkage, and independently each n = 0 to 5.

4. The amphiphilic copolymer of claim 1 or 2, said amphiphilic copolymer having a structure: 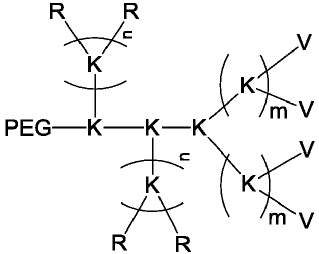 wherein PEG represents said polyethylene glycol, V represents a given vitamin E unit, K represents a first divalent linkage, R represents a crosslinking or cationic moiety, independently each m = 0 to 5, and independently each n = 0 to 20.

5. The amphiphilic copolymer of claim 1 or 2, said amphiphilic copolymer having a structure: 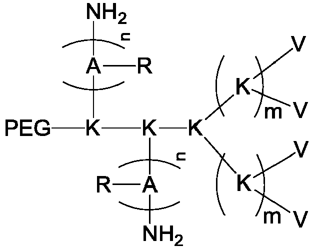 wherein PEG represents said polyethylene glycol, V represents a given vitamin E unit, K represents a first divalent linkage, A represents a second divalent linkage, R represents a crosslinking or cationic moiety, independently each m = 0 to 5, and independently each n = 0 to 20.

6. The amphiphilic copolymer of claim 1 or 2, said amphiphilic copolymer having a structure: 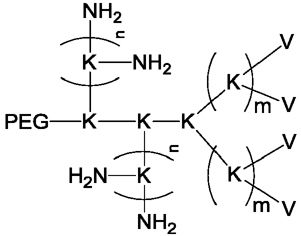 wherein PEG represents said polyethylene glycol, V represents a given vitamin E unit, K represents a first divalent linkage, independently each m = 0 to 5, and independently each n = 0 to 20.

7. The amphiphilic copolymer of claim 1 or 2, said amphiphilic copolymer having a structure: 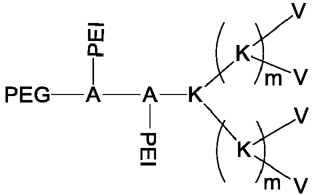 wherein PEG represents said polyethylene glycol, V represents a given vitamin E unit, K represents a first divalent linkage, A represents a second divalent linkage, PEI represents a polyethylenimine unit, and independently each m = 0 to 5.

8. The amphiphilic copolymer of any one of claims 3 to 7, wherein each of said one or more divalent linkages independently comprises lysine, N_{α}-Fmoc-N_{ε}-Boc-L-lysine, or aspartic acid.

9. The amphiphilic copolymer of any one of claims 5, 7 and 8, wherein each A comprises aspartic acid or β-benzyl-L-aspartate N-carboxy anhydride.

10. The amphiphilic copolymer of any one of claims 4 to 5 and 8 to 9, wherein each R independently comprises thioctic acid, cysteine, diethylenetriamine, triethylenetetramine, tris(2-aminoethyl)amine or N,N-diisopropylethylenediamine.

11. A nanocarrier comprising one or more amphiphilic copolymers, wherein each amphiphilic copolymer independently comprises the amphiphilic copolymer of any one of claims 1 to 10.

12. A composition comprising (i)(a) one or more amphiphilic copolymers, wherein each amphiphilic copolymer of said composition independently comprises the amphiphilic copolymer of any one of claims 1 to 10 or (i)(b) the nanocarrier of claim 11, and (ii) at least one biologically active substance combined with the one or more amphiphilic copolymers or the nanocarrier.

13. The composition of claim 12, wherein the at least one biologically active substance comprises a drug, an anti-carcinogenic compound, a nucleic acid, another small molecule, or any combination thereof.

14. The composition of claim 12 or 13, further comprising one or more additional components selected from the group consisting of sodium phosphate, sodium chloride, glucose, HEPES, mannitol, and combinations thereof.

15. The amphiphilic copolymer of any one of claims 1 to 10, the nanocarrier of claim 11, or the composition of any one of claims 12 to 14 for use for delivering one or more drugs, wherein the amphiphilic copolymer of any one of claims 1 to 10, the nanocarrier of claim 11, or the composition of any one of claims 12 to 14 is administered in an effective amount to the patient.

## Patentansprüche

1. Amphiphiles Copolymer, umfassend:
Polyethylenglykol und
drei oder mehr Vitamin E-Einheiten, die an das Polyethylenglykol gebunden sind.

2. Amphiphiles Copolymer nach Anspruch 1, worin das amphiphile Copolymer von 4 bis 8 Vitamin E-Einheiten, die an das Polyethylenglykol gebunden sind, umfasst.

3. Amphiphiles Copolymer nach Anspruch 1 oder 2, wobei das amphiphile Copolymer eine Struktur: 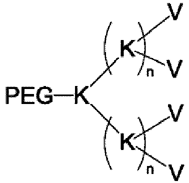 aufweist, worin PEG das Polyethylenglykol darstellt, V eine gegebene Vitamin E-Einheit darstellt, K eine erste divalente Bindung darstellt und unabhängig jedes n = 0 bis 5.

4. Amphiphiles Copolymer nach Anspruch 1 oder 2, wobei das amphiphile Copolymer eine Struktur: 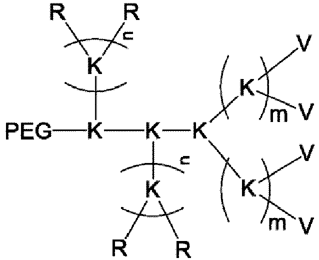 aufweist, worin PEG das Polyethylenglykol darstellt, V eine gegebene Vitamin E-Einheit darstellt, K eine erste divalente Bindung darstellt, R einen quervernetzenden oder kationischen Rest darstellt, unabhängig jedes m = 0 bis 5 und unabhängig jedes n = 0 bis 20.

5. Amphiphiles Copolymer nach Anspruch 1 oder 2, wobei das amphiphile Copolymer eine Struktur: 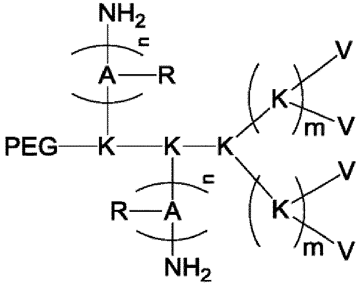 aufweist, worin PEG das Polyethylenglykol darstellt, V eine gegebene Vitamin E-Einheit darstellt, K eine erste divalente Bindung darstellt, A eine zweite divalente Bindung darstellt, R einen quervernetzenden oder kationischen Rest darstellt, unabhängig jedes m = 0 bis 5 und unabhängig jedes n = 0 bis 20.

6. Amphiphiles Copolymer nach Anspruch 1 oder 2, wobei das amphiphile Copolymer eine Struktur: 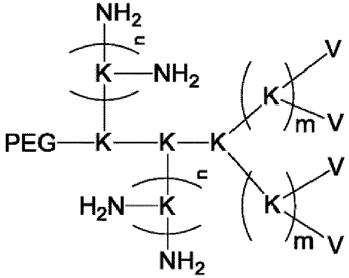 aufweist, worin PEG das Polyethylenglykol darstellt, V eine gegebene Vitamin E-Einheit darstellt, K eine erste divalente Bindung darstellt, unabhängig jedes m = 0 bis 5 und unabhängig jedes n = 0 bis 20.

7. Amphiphiles Copolymer nach Anspruch 1 oder 2, wobei das amphiphile Copolymer eine Struktur: 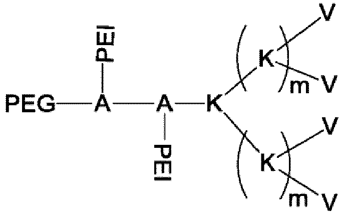 aufweist, worin PEG das Polyethylenglykol darstellt, V eine gegebene Vitamin E-Einheit darstellt, K eine erste divalente Bindung darstellt, A eine zweite divalente Bindung darstellt, PEI eine Polyethylenimineinheit darstellt, und unabhängig jedes m = 0 bis 5.

8. Amphiphiles Copolymer nach einem der Ansprüche 3 bis 7, worin jede der einen oder mehreren divalenten Bindungen unabhängig Lysin, N_{α}-Fmoc-N_{ε}-Boc-L-Lysin oder Asparaginsäure umfasst.

9. Amphiphiles Copolymer nach einem der Ansprüche 5, 7 und 8, worin jedes A Asparaginsäure oder ß-Benzyl-L-aspartat-N-carboxyanhydrid umfasst.

10. Amphiphiles Copolymer nach einem der Ansprüche 4 bis 5 und 8 bis 9, worin jedes R unabhängig Thioctsäure, Cystein, Diethylentriamin, Triethylentetramin, Tris(2-aminoethylamin) oder N,N-Diisopropylethylendiamin umfasst.

11. Nanoträger, umfassend ein oder mehrere amphiphile Copolymere, worin jedes amphiphile Copolymer unabhängig das amphiphile Copolymer nach einem der Ansprüche 1 bis 10 umfasst.

12. Zusammensetzung, umfassend (i)(a) ein oder mehrere amphiphile Copolymere, worin jedes amphiphile Copolymer dieser Zusammensetzung unabhängig das amphiphile Copolymer nach einem der Ansprüche 1 bis 10 umfasst oder (i)(b) den Nanoträger nach Anspruch 11 und (ii) mindestens eine biologisch aktive Substanz, kombiniert mit einem oder mehreren amphiphilen Copolymeren oder dem Nanoträger.

13. Zusammensetzung nach Anspruch 12, worin die mindestens eine biologisch aktive Substanz ein Arzneimittel, eine anikarzinogene Verbindung, eine Nukleinsäure, ein anderes kleines Molekül oder eine beliebige Kombination davon umfasst.

14. Zusammensetzung nach Anspruch 12 oder 13, weiterhin umfassend eine oder mehrere zusätzliche Komponenten, ausgewählt aus der Gruppe, bestehend aus Natriumphosphat, Natriumchlorid, Glukose, HEPES, Mannitol und Kombinationen davon.

15. Amphiphiles Copolymer nach einem der Ansprüche 1 bis 10, Nanoträger nach Anspruch 11 oder Zusammensetzung nach einem der Ansprüche 12 bis 14 zur Verwendung zur Zuführung von einem oder mehreren Arzneimitteln, worin das amphiphile Copolymer nach einem der Ansprüche 1 bis 10, der Nanoträger nach Anspruch 11 oder die Zusammensetzung nach einem der Ansprüche 12 bis 14 in einer wirksamen Menge dem Patienten verabreicht wird.

## Revendications

1. Copolymère amphiphile comprenant:
du polyéthylèneglycol et
trois ou plus de trois unités de vitamine E liées au polyéthylèneglycol.

2. Copolymère amphiphile selon la revendication 1, où ledit copolymère amphiphile comprend de quatre à huit unités de vitamine E liées au polyéthylèneglycol.

3. Copolymère amphiphile selon la revendication 1 ou 2, ledit copolymère amphiphile ayant une structure: 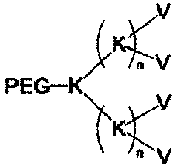 où PEG représente ledit polyéthylèneglycol, V représente une unité de vitamine E donnée, K représente un premier groupe de liaison divalent et indépendamment chaque n = 0 à 5.

4. Copolymère amphiphile selon la revendication 1 ou 2, ledit copolymère amphiphile ayant une structure: 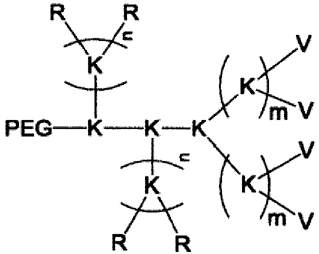 où PEG représente ledit polyéthylèneglycol, V représente une unité de vitamine E donnée, K représente un premier groupe de liaison divalent, R représente un groupement qui se réticule ou cationique, indépendamment chaque m = 0 à 5 et indépendamment chaque n = 0 à 20.

5. Copolymère amphiphile selon la revendication 1 ou 2, ledit copolymère amphiphile ayant une structure: 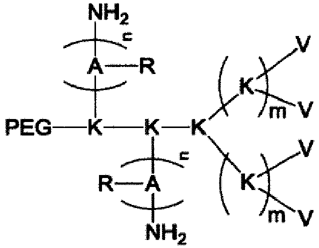 où PEG représente ledit polyéthylèneglycol, V représente une unité de vitamine E donnée, K représente un premier groupe de liaison divalent, A représente un second groupe de liaison divalent, R représente un groupement qui se réticule ou cationique, indépendamment chaque m = 0 à 5 et indépendamment chaque n = 0 à 20.

6. Copolymère amphiphile selon la revendication 1 ou 2, ledit copolymère amphiphile ayant une structure: 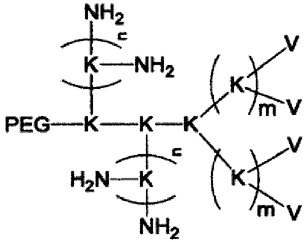 où PEG représente ledit polyéthylèneglycol, V représente une unité de vitamine E donnée, K représente un premier groupe de liaison divalent, indépendamment chaque m = 0 à 5 et indépendamment chaque n = 0 à 20.

7. Copolymère amphiphile selon la revendication 1 ou 2, ledit copolymère amphiphile ayant une structure: 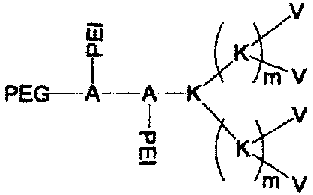 où PEG représente ledit polyéthylèneglycol, V représente une unité de vitamine E donnée, K représente un premier groupe de liaison divalent, A représente un second groupe de liaison divalent, PEI représente une unité de polyéthylèneimine, et indépendamment chaque m = 0 à 5.

8. Copolymère amphiphile selon l'une quelconque des revendications 3 à 7, où chacun desdits un ou plusieurs groupes de liaison divalents comprend indépendamment de la lysine, N_{α}-Fmoc-N_{ε}-Boc-L-lysine ou de l'acide aspartique.

9. Copolymère amphiphile selon l'une quelconque des revendications 5, 7 et 8, où chaque A comprend de l'acide aspartique ou du N-carboxyanhydride de β-benzyl-L-aspartate.

10. Copolymère amphiphile selon l'une quelconque des revendications 4 à 5 et 8 à 9, où chaque R comprend indépendamment de l'acide thioctique, de la cystéine, de la diéthylènetriamine, de la triéthylènetétramine, de la tris(2-amino-éthyl)amine ou de la N,N-diisopropyléthylènediamine.

11. Nanovecteur comprenant un ou plusieurs copolymères amphiphiles, où chaque copolymère amphiphile comprend indépendamment le copolymère amphiphile selon l'une quelconque des revendications 1 à 10.

12. Composition comprenant (i) (a) un ou plusieurs copolymères amphiphiles, où chaque copolymère amphiphile de ladite composition comprend indépendamment le copolymère amphiphile selon l'une quelconque des revendications 1 à 10 ou (i) (b) le nanovecteur selon la revendication 11, et (ii) au moins une substance biologiquement active combinée avec les un ou plusieurs copolymères amphiphiles ou le nanovecteur.

13. Composition selon la revendication 12, où la au moins une substance biologiquement active comprend un médicament, un composé anticarcinogène, un acide nucléique, une autre petite molécule ou toute combinaison de ceux-ci.

14. Composition selon la revendication 12 ou 13, comprenant en outre un ou plusieurs composants supplémentaires choisis dans le groupe consistant en le phosphate de sodium, le chlorure de sodium, le glucose, HEPES, le mannitol et leurs combinaisons.

15. Copolymère amphiphile selon l'une quelconque des revendications 1 à 10, nanovecteur selon la revendication 11 ou composition selon l'une quelconque des revendications 12 à 14 destiné(e) à être utilisé(e) pour délivrer un ou plusieurs médicaments, où le copolymère amphiphile selon l'une quelconque des revendications 1 à 10, le nanovecteur selon la revendication 11 ou la composition selon l'une quelconque des revendications 12 à 14 est administré(e) en une quantité efficace au patient.
